(19) Europäisches Patentamt
European Patent Office
Office européen des brevets

(11) EP 3 936 142 B1

(12) EUROPEAN PATENT SPECIFICATION

(45) Date of publication and mention of the grant of the patent:
**17.01.2024 Bulletin 2024/03**

(21) Application number: **20832900.3**

(22) Date of filing: **29.06.2020**

(51) International Patent Classification (IPC):
***A61K 38/17*** *(2006.01)* ***A61P 1/16*** *(2006.01)*
***A61K 47/68*** *(2017.01)* ***A61P 29/00*** *(2006.01)*
***A61P 35/00*** *(2006.01)*

(52) Cooperative Patent Classification (CPC):
**A61K 38/1796; A61K 47/68; A61K 47/6811; A61P 29/00; A61P 35/00;** A61P 1/16

(86) International application number:
**PCT/KR2020/008479**

(87) International publication number:
**WO 2020/263063 (30.12.2020 Gazette 2020/53)**

(54) **TRIPLE AGONIST HAVING ACTIVITY WITH RESPECT TO ALL OF GLUCAGON, GLP-1, AND GIP RECEPTORS FOR TREATING LIVER DISEASE**

TRIPLE-AGONISTEN MIT AKTIVITÄT IN BEZUG AUF ALLE GLUCAGON-, GLP-1- UND GIP-REZEPTOREN ODER ZUR BEHANDLUNG VON LEBERERKRANKUNG

TRIPLE AGONISTE AYANT UNE ACTIVITÉ PAR RAPPORT À TOUS LES RÉCEPTEURS DU GLUCAGON, DE GLP-1 ET DE GIP POUR TRAITEMENT DE MALADIES HÉPATHIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.06.2019 KR 20190077776**
**13.01.2020 KR 20200004386**
**13.01.2020 KR 20200004379**
**08.06.2020 KR 20200069219**

(43) Date of publication of application:
**12.01.2022 Bulletin 2022/02**

(60) Divisional application:
**23215168.8**

(73) Proprietor: **Hanmi Pharm. Co., Ltd.**
**Hwaseong-si, Gyeonggi-do 18536 (KR)**

(72) Inventors:
- **KWON, Hyun Joo**
  **Hwaseong-si, Gyeonggi-do 18469 (KR)**
- **KIM, Jung Kuk**
  **Hwaseong-si, Gyeonggi-do 18469 (KR)**
- **PARK, Eun Jin**
  **Hwaseong-si, Gyeonggi-do 18469 (KR)**
- **LEE, Jong Min**
  **Hwaseong-si, Gyeonggi-do 18469 (KR)**
- **LEE, Jong Suk**
  **Hwaseong-si, Gyeonggi-do 18469 (KR)**
- **JO, Hyo Sang**
  **Hwaseong-si, Gyeonggi-do 18469 (KR)**
- **CHOI, In Young**
  **Hwaseong-si, Gyeonggi-do 18469 (KR)**

(74) Representative: **Mewburn Ellis LLP**
**Aurora Building**
**Counterslip**
**Bristol BS1 6BX (GB)**

(56) References cited:
**US-A1- 2018 311 315**

- **KIM JUNG KUK ET AL: "Therapeutic Effect of a Novel Long-Acting GLP-1/GIP/Glucagon Triple Agonist (HM15211) in a NASH and Fibrosis Animal Model", DIABETES, vol. 68, no. Suppl. 1, 1 June 2019 (2019-06-01), pages 990-P, XP9540757, & 79TH SCIENTIFIC SESSIONS OF THE AMERICAN-DIABETES-ASSOCIATION (ADA); SAN FRANCISCO, CA, USA; JUNE 07 -11, 2019**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **CHOI IN YOUNG ET AL: "Potent Body Weight Loss and Efficacy in a NASH Animal Model by a Novel Long-Acting GLP-1/Glucagon/GIP Tri-Agonist (HM15211)", DIABETES, vol. 66, no. Suppl. 1, 1 June 2017 (2017-06-01), page A304, XP9540760, & 77TH SCIENTIFIC SESSIONS OF THE AMERICAN-DIABETES-ASSOCIATION; SAN DIEGO, CA, USA; JUNE 09 -13, 2017**
- **CHOI IN YOUNG ET AL: "Effect of a Novel Long-Acting GLP-1/GIP/Glucagon Triple Agonist (HM15211) in a NASH and Fibrosis Animal Model", DIABETES, vol. 67, no. Suppl. 1, 1 July 2018 (2018-07-01), XP9540759, & 78TH SCIENTIFIC SESSIONS OF THE AMERICAN-DIABETES-ASSOCIATION; ORLANDO, FL, USA; JUNE 22 -26, 2018**
- **KIM J ET AL: "Therapeutic effect of a novel long-acting GLP-1/GIP/Glucagon triple agonist (HM15211) in NASH and fibrosis animal models", DIABETOLOGIA, vol. 61, no. Suppl. 1, October 2018 (2018-10), pages S61-S62, XP9540758, & 54TH ANNUAL MEETING OF THE EUROPEAN-ASSOCIATION-FOR-THE-STUDY-OF-DIAB ETES (EASD); BERLIN, GERMANY; OCTOBER 01 -05, 2018**
- **In Young Choi, Jung Kuk Kim, Jong Suk Lee, Eun Jin Park, Dae Jin Kim, Young Hoon Kim, and Sun Jin Kim: "Effect of a novel long-acting GLP-1/GIP/Glucagon triple agonist (HM15211) in a NASH and fibrosis animal model", hanmipharm.com , 22 June 2018 (2018-06-22), XP002808047, Retrieved from the Internet: URL:https://www.hanmipharm.com/ehanmi/rnd/HM15211/7.2018_ADA_Poster_HM15211.pdf [retrieved on 2022-11-21]**
- **Jung Kuk Kim1, Jong Suk Lee1, Dae Jin Kim1, Eun Jin Park1, Aram Lee1 , Young Hoon Kim1, and In Young Choi: "Therapeutic effect of a novel long-acting GLP-1/GIP/Glucagon triple agonist (HM15211) ina NASH and fibrosis animal model", hanmipharm.com , 11 June 2019 (2019-06-11), XP002808048, Retrieved from the Internet: URL:https://www.hanmipharm.com/ehanmi/rnd/HM15211/13.2019_ADA_Poster_HM15211.pdf [retrieved on 2022-11-21]**


Remarks:

The complete document including Reference Table(s) and the Sequence Listing(s) can be downloaded from the EPO website

## Description

### [Technical Field]

The present invention relates to therapeutic uses of a triple agonist having activities to all of glucagon, GLP-1, and GIP receptors, or conjugates thereof for liver disease.

### [Background Art]

The liver is one of the major organs in the living body of an animal, and representative examples of liver-related diseases include non-alcoholic fatty liver (NAFL), hepatitis, liver fibrosis, cholestasis liver disease, cirrhosis, liver decompensation, liver cancer, *etc.* It is known that viruses, alcohol, drugs, immune abnormalities, metabolic diseases, *etc.* can cause inflammation of the liver, and diseases such as liver fibrosis, cirrhosis, liver cancer, *etc.* can develop as the liver inflammation progresses and becomes chronic.

In general, hepatitis, which is a disease characterized by inflammation of the liver, accounts for most liver diseases, and it is known that as hepatitis progresses, various liver diseases (*e.g.*, liver fibrosis, cirrhosis, *etc.*) may appear accompanied by liver inflammation or caused by liver inflammation. Hepatitis can be divided into acute hepatitis and chronic hepatitis according to its features, and it can be divided into viral hepatitis, alcoholic hepatitis, and drug hepatitis according to its causes. Cholestasis liver disease is also assumed to be caused by inflammatory disease.

Other representative examples of liver disease include metabolic liver disease (*e.g.*, fatty liver, non-alcoholic fatty liver disease (NAFLD; non-alcoholic steatohepatitis), steatohepatitis, liver fibrosis, cirrhosis, liver decompensation, liver cancer, *etc.* Since these liver diseases can only be discovered after considerable progress due to the absence of any symptoms or awareness thereof in the early stages, they rank as leading causes of death not only in Korea but also worldwide, and thus, there is a large demand for the development of therapeutic drugs.

Liver fibrosis is a result of a wound recovery process for repeated liver damage, and normal recovery may be possible when the cause of the liver damage is removed, but cirrhosis occurs when liver fibrosis is repeated and fibrosis is aggravated. Cirrhosis is a chronic disease, which is pathologically accompanied by necrosis, inflammation, and fibrosis of liver cells, and which develops into diseases, such as cirrhosis complications (*e.g.*, liver decompensation), liver cancer, *etc.,* eventually leading to death. In particular, since cirrhosis can be discovered only after considerable progress due to the absence of awareness of one's symptoms in the early stages of the disease, studies are actively underway to develop a method for prompt treatment of liver fibrosis, which is a condition before it evolves into cirrhosis, *etc.* Dr. Kunos's team recently reported a drug which is developed by chemically improving ibipinapant, which is a type of brain-penetrating cannabinoid type 1 (CB-1) receptor antagonist, but it is still unclear whether this drug is effective as a real drug (JCI Insight. 2016; 1(11): e87336.doi: 10.1 1 72/j ci.insight. 87336). Accordingly, there is still a need for the development of a drug capable of treating fibrosis of various tissues or fibrosis of the liver that can provide patient convenience without side effects.

Non-alcoholic steatohepatitis disease (NAFLD), a metabolic liver disease, is a disease that shows tissue findings similar to alcoholic hepatitis despite not being related to alcohol consumption, and it includes simple steatosis, non-alcoholic fatty liver (NAFL), non-alcoholic steatohepatitis (NASH), *etc.* Non-alcoholic steatohepatitis disease (NAFLD) has shown an increasing trend along with the increase in obesity and diabetes populations, and the annual incidence rate in Korea is about 16%.

In order to prevent and/or treat such non-alcoholic steatohepatitis disease, efforts are being made to improve insulin resistance. For example, clinical trials on thiazolidinediones (TZDs) or metformin, which is a type of insulin sensitizer, are still actively underway (Hepatology (2003) 38: 1008-17, J Clin Invest. (2001) 108: 1167-74).

However, in the case of treatment using the TZD-based drugs, they have disadvantages in that they may cause a large weight gain and reduce the flow rate of bodily fluids. Therefore, the application of these drugs has been known to be impossible for patients with heart disease. Due to these results, *etc.,* it has been variously known in the art that the direct use of drugs which are known to be effective in treating diabetes (*e.g.*, insulin resistance improvers) as a therapeutic agent for the treatment of non-alcoholic steatohepatitis disease (NAFLD) may cause problems such as side effects.

Meanwhile, it is known that macrophages are responsible for important immune responses in the liver and are involved in non-alcoholic steatohepatitis disease (NAFLD) including non-alcoholic steatohepatitis (NASH) (Nat Rev Gastroenterol Hepatol. 2019 Mar; 16(3): 145-159). Specifically, it is known that macrophages are activated in patients with non-alcoholic steatohepatitis disease (NAFLD), and that drugs targeting these macrophages can inhibit inflammation and fibrosis in the liver and show therapeutic efficacy against non-alcoholic steatohepatitis (NASH).

Glucagon-like peptide-1 (GLP-1) and glucose-dependent insulinotropic polypeptide (GIP) are representative gastrointestinal hormones and neurohormones involved in the regulation of blood glucose components according to food intake. Glucagon is a peptide hormone secreted by the pancreas and is involved in the regulation of blood glucose

levels along with the two materials described above.

GLP-1 is a hormone secreted by the small intestine and is stimulated by food intake, and it promotes insulin secretion in the pancreas in a blood glucose-dependent manner and inhibits the secretion of glucagon, thus helping the action of lowering blood glucose levels. Additionally, GLP-1 has the roles of slowing digestive action in the gastrointestinal tract by acting as a satiety factor and reducing the amount of food intake by delaying the time for digested food to pass through the gastrointestinal tract. Moreover, it was reported that the administration of GLP-1 to mice has effects of inhibiting food intake and reducing body weight, and these effects were confirmed to occur equally in both normal and obese states, thus showing the potential of GLP-1 as a therapeutic agent for treating obesity.

GIP, being one of the gastrointestinal hormones secreted by the stimulation of food intake like GLP-1, is a hormone consisting of 42 amino acids secreted by the intestinal K-cells. It was reported that GIP performs the functions of promoting insulin secretion in the pancreas in a blood glucose-dependent manner and helping lower the blood glucose levels, has the effect of increasing the activation of GLP-1, *etc.*

Glucagon is produced in the pancreas when the blood glucose levels fall due to reasons such as medication, disease, deficiency in hormones or enzymes, *etc.* Glucagon sends a signal for glycogen breakdown in the liver to induce the release of glucose and thereby increases blood glucose levels to a normal level. In addition to the effect of increasing the blood glucose levels, glucagon has been reported to suppress appetite in animals and humans and activate hormone-sensitive lipase of adipocytes so as to promote lipolysis and energy expenditure, thereby showing an anti-obesity effect.

Kim et al (Diabetes 2019;68(Supplement_1):990-P) relates to the "therapeutic effect of a novel long-acting GLP-1/GIP/Glucagon triple agonist (HM15211) in a NASH and fibrosis animal model".

Choi et al (Diabetes 2017;66(Supplement_1):A304) relates to "potent body weight loss and efficacy in a NASH animal model by a novel long-acting GLP-1/Glucagon/GIP tri-agonist (HM15211)".

Choi et al (Diabetes 2018;67(Supplement_1):1106-P) relates to the "effect of a novel long-acting GLP-1/GIP/Glucagon triple agonist (HM15211) in a NASH and fibrosis animal model".

Kim et al (Diabetologia 2018; 61(Supplement 1):S61-S62 relates to the "therapeutic effect of a novel long-acting GLP-1/GIP/Glucagon triple agonist (HM15211) in a NASH and fibrosis animal models".

Choi et al (2018; https://www.hanmipharm.com/ehanmi/rnd/HM15211/7.2018_ADA_Poster_HM15211.pdf) relates to "effect of a novel long-acting GLP-1/GIP/Glucagon triple agonist (HM15211) in a NASH and fibrosis animal model".

Kim et al (2019; https://www.hanmipharm.com/ehanmi/rnd/HM15211/13.2019_ADA_Poster_HM15211.pdf) relates to "therapeutic effect of a novel long-acting GLP-1/GIP/Glucagon triple agonist (HM15211) in a NASH and fibrosis animal model".

US 2018/311315 A1 relates to "a long acting conjugate of a triple agonist having activities to all of glucagon, GLP-1, and GIP receptors".

**[Disclosure]**

**[Technical Problem]**

An object of the present invention is to provide a pharmaceutical composition for use in the prevention or treatment of liver disease, which contains a peptide that has activities to a glucagon receptor, a glucagon-like peptide-1 (GLP-1) receptor, and a glucose-dependent insulinotropic polypeptide (GIP) receptor, or a conjugate thereof.

Also disclosed herein but not part of the claimed invention is a method for the prevention or treatment of liver disease, which includes administering the peptide or a composition containing the peptide to a subject in need thereof.

Also disclosed herein but not part of the claimed invention is use of the peptide or a composition containing the peptide in the preparation of a medicament for the prevention or treatment of liver disease.

**[Technical Solution]**

The present invention is as defined in the claims. This invention and other facets of the disclosure may be more fully understood by reference to the following description.

To achieve the above objects, the present disclosure provides a pharmaceutical composition for use in the prevention or treatment of liver disease, which contains a peptide that has activities to a glucagon receptor, a glucagon-like peptide-1 (GLP-1) receptor, and a glucose-dependent insulinotropic polypeptide (GIP) receptor, or a conjugate thereof.

In the claimed invention, the pharmaceutical composition comprises a pharmaceutically acceptable excipient and a peptide comprising an amino acid sequence of SEQ ID NO: 42 in a pharmaceutically effective amount. The liver disease is at least one disease selected from the group consisting of simple steatosis, non-alcoholic fatty liver (NAFL), liver inflammation, non-alcoholic steatohepatitis (NASH), metabolic liver disease, cholestatic liver disease, liver fibrosis,

liver cirrhosis, liver decompensation, and liver cancer.

In disclosed but not explicitly claimed cases, the pharmaceutical composition for the prevention or treatment of liver disease contains a pharmaceutically acceptable excipient; and a peptide containing an amino acid sequence of any one of SEQ ID NOS: 1 to 102 in a pharmaceutically effective amount.

In some cases, the peptide is characterized in that it is in the form of a long-acting conjugate, and the long-acting conjugate is characterized in that it is represented by the following Formula 1:

[Formula 1] X-L-F

wherein, in Formula 1 above,

X is a peptide of an amino acid sequence of SEQ ID NO: 42;
L is a linker including an ethylene glycol repeat unit;
F is an immunoglobulin Fc fragment or a derivative thereof; and
'-' represents a covalent bond between X and L and between L and F.

In some cases, the peptide is characterized in that the C-terminus of the peptide is amidated.

In some cases, the liver disease is characterized in that it is liver inflammation.

In some cases, the pharmaceutical composition is characterized in that it reduces the expression of at least one of TNF-$\alpha$, MCP-1, and IL-6 in the liver tissue when administered.

In some cases, the liver disease is characterized in that it is a metabolic liver disease.

In some cases, the pharmaceutical composition is characterized in that it reduces the amount of triglycerides and/or cholesterol in the liver tissue when administered.

In some cases, the liver disease is characterized in that it is at least one disease selected from the group consisting of simple steatosis, non-alcoholic fatty liver (NAFL), liver inflammation, non-alcoholic steatohepatitis (NASH), cholestasis liver disease, liver fibrosis, cirrhosis, liver decompensation, and liver cancer.

In some cases, the cholestasis liver disease is characterized in that it is any one selected from the group consisting of primary biliary cirrhosis, primary sclerosing cholangitis, and a combination thereof.

In some cases, the liver disease is characterized in that it is non-alcoholic steatohepatitis (NASH) that is accompanied by fatty liver, liver fibrosis, or cirrhosis.

In some cases, the liver disease is characterized in that it is liver cancer caused by non-alcoholic steatohepatitis (NASH).

In some cases, the liver disease is characterized in that it is at least one disease selected from the group consisting of simple steatosis, non-alcoholic fatty liver (NAFL), and cirrhosis.

In some cases, the liver disease is characterized in that it is at least one disease selected from the group consisting of liver inflammation, non-alcoholic steatohepatitis (NASH), and liver fibrosis.

In some cases, the liver disease is characterized in that it is liver fibrosis, and the pharmaceutical composition is characterized in that it reduces the blood concentration of TIMP-1 and/or hyaluronic acid in a subject administered with the pharmaceutical composition when administered.

In some disclosed but not explicitly claimed cases, the peptide is characterized in that it includes an amino acid sequence selected from the group consisting of SEQ ID NOS: 21, 22, 43, 50, 64, 66, 67, 70, 71, 76, 77, 96, 97, and 100.

In embodimentssome disclosed but not explicitly claimed cases, the peptide is characterized in that it includes an amino acid sequence selected from the group consisting of SEQ ID NOS: 21, 22, 43, 50, 66, 67, 77, 96, 97, and 100.

In some disclosed but not explicitly claimed cases, the peptide is characterized in that it includes an amino acid sequence selected from the group consisting of SEQ ID NOS: 21, 22, 43, 50, 77, and 96.

In some cases, the formula weight of the ethylene glycol repeat unit portion in the L is in the range of 1 kDa to 100 kDa.

Also disclosed herein but part of the claimed invention is a method for the prevention or treatment of liver disease, which includes administering the peptide or a composition containing the peptide to a subject in need thereof.

Also disclosed herein but part of the claimed invention is use of the peptide or a composition containing the peptide in the manufacture of a medicament for the prevention or treatment of liver disease.

**[Advantageous Effects]**

The triple agonist or conjugate thereof according to the present disclosure can have a use for the prevention or treatment of liver disease.

**[Brief Description of Drawings]**

FIG. 1 shows a graph illustrating the results of changes in NAFLD activity score (NAS) in mice by administering the long-acting conjugate of SEQ ID NO: 42 once every 2 days for 28 days to a mouse model of non-alcoholic steatohepatitis (NASH) induced by methionine- and choline-deficient (MCD) dietary intake (p<0.05, **p<0.01, ***p<0.001, *vs.* vehicle by one-way ANOVA).

FIG. 2 shows a graph illustrating the results of confirming the effect of improving fatty liver by the long-acting conjugate of SEQ ID NO: 42 in mice with steatohepatitis induced by AMI,N diet.

FIG. 3 shows a graph and images illustrating the results of confirming the effect of reducing the steatosis score by the long-acting conjugate of SEQ ID NO: 42 in mice with steatohepatitis induced by AMLN diet.

FIG. 4 shows a graph illustrating the results of changes in ELF score according to the administration of the long-acting conjugate of SEQ ID NO: 42 in a mouse model of liver fibrosis induced by TAA administration (*p<0.05, **p<0.01, ***p<0.001, *vs.* vehicle by one-way ANOVA).

FIG. 5 shows a graph illustrating the changes in the sirius red staining positive area in liver tissue according to the administration of the long-acting conjugate of SEQ ID NO: 42 in a mouse model of liver fibrosis induced by TAA administration (*p<0.05, **p<0.01, ***p<0.001, *vs*. vehicle by one-way ANOVA).

FIG. 6 shows graphs illustrating the changes in concentration of a liver fibrosis marker in the blood according to the administration of the long-acting conjugate of SEQ ID NO: 42 in a mouse model of liver fibrosis induced by BDL (*p<0.05, **p<0.01, ***p<0.001, *vs*. vehicle by one-way ANOVA, $^{\dagger\dagger\dagger}$p<0.01 *vs*. obeticholic acid by unpaired t-test).

FIG. 7a shows images illustrating the results of sirius red staining according to the administration of the long-acting conjugate of SEQ ID NO: 42 in a mouse model of liver fibrosis induced by BDL.

FIG. 7b shows a graph illustrating the fibrosis score of liver tissue according to the administration of the long-acting conjugate of SEQ ID NO: 42 in a mouse model of liver fibrosis induced by BDL (*p<0.05, **p<0.01, ***p<0.001, *vs.* vehicle by one-way ANOVA).

FIG. 8 shows images illustrating the results of H&E staining and a graph illustrating the changes in inflammation score of liver tissue according to the administration of the long-acting conjugate of SEQ ID NO: 42 in a primary biliary cirrhosis (PBC) mouse model (*p<0.05, **p<0.01, ***p<0.001, *vs.* vehicle by one-way ANOVA).

FIG. 9 shows images illustrating the results of H&E staining and a graph illustrating the changes in parenchymal necrosis score of liver tissue according to the administration of the long-acting conjugate of SEQ ID NO: 42 in a primary sclerosing cholangitis (PSC) mouse model (*p<0.05, **p<0.01, ***p<0.001, *vs.* vehicle by one-way ANOVA).

FIG. 10 shows a graph illustrating the changes in bile duct hyperplasia score according to the administration of the long-acting conjugate of SEQ ID NO: 42 in the PSC mouse model (*p<0.05, **p<0.01, ***p<0.001, *vs.* vehicle by one-way ANOVA).

FIG. 11 shows graphs illustrating the changes in inflammation-related cytokine expression level in liver tissue according to the administration of the long-acting conjugate of SEQ ID NO: 42 (*p<0.05, **p<0.01, ***p<0.001, *vs.* vehicle by one-way ANOVA).

FIG. 12 shows a graph illustrating the results of confirming the effect of reducing the human tumor necrosis factor-α (TNF-α) by the triple agonists of SEQ ID NOS: 42, 66, 67, 97, and 100 in a human macrophage cell line.

**[DETAILED DESCRIPTION]**

Hereinafter, the present invention and other facets of the disclosure will be described in more detail.

Meanwhile, each of the explanations and exemplary embodiments disclosed herein can be applied to each other explanation and exemplary embodiment.

Throughout the entire specification, not only the conventional one-letter and three-letter codes for naturally occurring amino acids, but also those three-letter codes generally allowed for other amino acids, such as α-aminoisobutyric acid (Aib), Sar (*N*-methylglycine), and α-methyl-glutamic acid, are used. Additionally, the amino acids mentioned herein are abbreviated according to the nomenclature rules of IUPAC-IUB as follows:

| | |
|---|---|
| alanine (Ala, A) | arginine (Arg, R) |
| asparagine (Asn, N) | aspartic acid (Asp, D) |
| cysteine (Cys, C) | glutamic acid (Glu, E) |
| glutamine (Gln, Q) | glycine (Gly, G) |
| histidine (His, H) | isoleucine (Ile, I) |
| leucine (Leu, L) | lysine (Lys, K) |

(continued)

| | |
|---|---|
| methionine (Met, M) | phenylalanine (Phe, F) |
| proline (Pro, P) | serine (Ser, S) |
| threonine (Thr, T) | tryptophan (Trp, W) |
| tyrosine (Tyr, Y) | valine (Val, V) |

To achieve the objects of the present disclosure, the present disclosure provides a pharmaceutical composition for preventing or treating liver disease, which contains a peptide having activities to a glucagon receptor, a glucagon-like peptide-1 (GLP-1) receptor, and a glucose-dependent insulinotropic polypeptide (GIP) receptor, and specifically, a peptide containing an amino acid sequence of any one of SEQ ID NOS: 1 to 102.

In the present disclosure, the "peptide having activities to a glucagon receptor, a GLP-1 receptor, and a GIP receptor" can be used interchangeably with a triple agonist.

Such a peptide includes various materials which have a significant level of activities to glucagon, GLP-1, and GIP receptors (*e.g*., various peptides).

The triple agonist having a significant level of activities to glucagon, GLP-1, and GIP receptors may exhibit *in vitro* activities which are about 0.001% or higher, about 0.01% or higher, about 0.1% or higher, about 1% or higher, about 2% or higher, about 3% or higher, about 4% or higher, about 5% or higher, about 6% or higher, about 7% or higher, about 8% or higher, about 9% or higher, about 10% or higher, about 20% or higher, about 30% or higher, about 40% or higher, about 50% or higher, about 60% or higher, about 70% or higher, about 80% or higher, about 90% or higher, and about 100% or higher, to one or more receptors, specifically two or more receptors, and more specifically all three of the receptors among the glucagon, GLP-1, and GIP receptors, compared to native ligands of the corresponding receptors (native glucagon, native GLP-1, and native GIP), but the triple agonist is not particularly limited thereto, and activity ranges with a significant increase are included without limitation.

In particular, the activities to receptors may include, for example, those cases where the *in vitro* activities are 0.1% or higher, 1% or higher, 2% or higher, 3% or higher, 4% or higher, 5% or higher, 6% or higher, 7% or higher, 8% or higher, 9% or higher, 10% or higher, 20% or higher, 30% or higher, 40% or higher, 50% or higher, 60% or higher, 70% or higher, 80% or higher, 90% or higher, 100% or higher, and about 200% or higher compared to native receptors, but the activities are not limited thereto.

As used herein, the term "about" refers to a range including all of $\pm$ 0.5, $\pm$ 0.4, $\pm$ 0.3, $\pm$ 0.2, $\pm$ 0.1, *etc.,* and it includes all of the values equivalent to those which come immediately after the term "about" or those in a similar range, but is not limited thereto.

The method for measuring the *in vitro* activity of the triple agonist may be referred to Example 1, but the method is not particularly limited thereto.

Meanwhile, the triple agonist is characterized by having activities of i) to iii) described below, and specifically a significant activity thereof:

i) activation of a GLP-1 receptor; ii) activation of a glucagon receptor; and iii) activation of a GIP receptor.

In particular, the activation of receptors may include, for example, those cases where the *in vitro* activities are about 0.1% or higher, about 1% or higher, about 2% or higher, about 3% or higher, about 4% or higher, about 5% or higher, about 6% or higher, about 7% or higher, about 8% or higher, about 9% or higher, about 10% or higher, about 20% or higher, about 30% or higher, about 40% or higher, about 50% or higher, about 60% or higher, about 70% or higher, about 80% or higher, about 90% or higher, and about 100% or higher, compared to native receptors, but the activities are not limited thereto.

Additionally, the peptide may be one which has an increased *in vivo* half-life compared to any one of native GLP-1, native glucagon, and native GIP, but the peptide is not particularly limited thereto.

The peptide may be a peptide which is not naturally occurring, but is not particularly limited thereto.

The peptide may be an analog of native glucagon, but is not particularly limited thereto. Specifically, the native glucagon analog includes peptides which have at least one difference in the amino acid sequence compared to that of native glucagon; peptides which are modified via modification of the native glucagon sequence; and mimetics of the native glucagon.

Meanwhile, native glucagon may have the following amino acid sequence, but is not particularly limited thereto:

His-Ser-Gln-Gly-Thr-Phe-Thr-Ser-Asp-Tyr-Ser-Lys-Tyr-Leu-Asp-Ser-Arg-Arg-Ala-Gln -Asp-Phe-Val-Gln-Trp-Leu-Met-Asn-Thr (SEQ ID NO: 118)

Specifically, the peptide may be an analog of native glucagon in which a variation selected from the group consisting of substitution, addition, deletion, modification, and a combination thereof has occurred on at least one amino acid of the native glucagon sequence, but the peptide is not particularly limited thereto.

Additionally, the substitution of an amino acid includes both a substitution with an amino acid and a substitution with a non-native compound.

Additionally, the addition may be performed at the N-terminus and/or C-terminus of a peptide. Meanwhile, the length of the amino acid to be added is not particularly limited, but 1 or more, 2 or more, 3 or more, 4 or more, 5 or more, 6 or more, 7 or more, 8 or more, 9 or more, 10 or more, and 11 or more amino acids may be added, and in a broad sense, the addition may include an addition of a polypeptide, but the addition is not particularly limited thereto.

More specifically, the peptide may be those where 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, or 20 amino acids selected from the group consisting of amino acids at positions 1, 2, 3, 7, 10, 12, 13, 14, 15, 16, 17, 18, 19, 20, 21, 23, 24, 27, 28, and 29 in the amino acid sequence of native glucagon are substituted with other amino acids, and in addition, may be those where 1 or more, 2 or more, 3 or more, 4 or more, 5 or more, 6 or more, 7 or more, 8 or more, 9 or more, 10 or more, or 11 or more amino acids are independently or additionally added to the C-terminus thereof, but the peptide is not particularly limited thereto.

Even more specifically, the peptide may be those where 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, or 19 amino acids selected from the group consisting of amino acids at positions 1, 2, 3, 10, 12, 13, 14, 15, 16, 17, 18, 19, 20, 21, 23, 24, 27, 28, and 29 in the amino acid sequence of native glucagon are substituted with other amino acids, and in addition, may be those where 1 or more, 2 or more, 3 or more, 4 or more, 5 or more, 6 or more, 7 or more, 8 or more, 9 or more, 10 or more, or 11 or more amino acids are independently or additionally added to the C-terminus thereof, but the peptide is not particularly limited thereto.

Even more specifically, the peptide may be those where 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, or 17 amino acids selected from the group consisting of amino acids at positions 1, 2, 3, 10, 13, 14, 15, 16, 17, 18, 19, 20, 21, 23, 24, 28, and 29 in the amino acid sequence of native glucagon are substituted with other amino acids, and in addition, may be those where 1 or more, 2 or more, 3 or more, 4 or more, 5 or more, 6 or more, 7 or more, 8 or more, 9 or more, 10 or more, or 11 or more amino acids are independently or additionally added to the C-terminus thereof, but the peptide is not particularly limited thereto.

Even more specifically, the peptide may be those where 1 or more, 2 or more, 3 or more, 4 or more, 5 or more, 6 or more, 7 or more, 8 or more, 9 or more, 10 or more, 11 or more, 12 or more, 13 or more, or 14 amino acids selected from the group consisting of amino acids at positions 1, 2, 13, 16, 17, 18, 19, 20, 21, 23, 24, 27, 28, and 29 in the amino acid sequence of native glucagon are substituted with other amino acids, and in addition, may be those where 1 or more, 2 or more, 3 or more, 4 or more, 5 or more, 6 or more, 7 or more, 8 or more, 9 or more, 10 or more, or 11 or more amino acids are independently or additionally added to the C-terminus thereof, but the peptide is not particularly limited thereto.

The amino acids to be introduced may be selected from the group consisting of tyrosine, $\alpha$-methyl-glutamic acid, Aib, methionine, glutamic acid, histidine, lysine, leucine, isoleucine, glutamine, valine, glycine, alanine, cysteine, serine, alanine, aspartic acid, and arginine, but the amino acids to be introduced are not particularly limited thereto.

For example, the amino acid sequence(s) to be added may be one or more amino acid sequences derived from a native GLP-1 amino acid sequence, a native GIP amino acid sequence, or a native exendin-4 amino acid sequence.

Such a peptide may include an intramolecular bridge (*e.g.,* a covalent crosslinking or non-covalent crosslinking), and specifically, may be in the form including a ring, for example, may be in the form where a ring is formed between the $16^{th}$ amino acid and the $20^{th}$ amino acid of the peptide, but the peptide is not particularly limited thereto.

A non-limiting example of the ring may include a lactam bridge (or a lactam ring).

Additionally, the peptide includes all of those which are modified to include a ring, or include an amino acid capable of forming a ring in a target position.

For example, the peptide may be one where one of the $16^{th}$ and $20^{th}$ amino acids is substituted with glutamic acid and the other with lysine, which can form a ring, but the peptide is not limited thereto.

Such a ring may be formed between amino acid side chains within the peptide; for example, the ring may be in the form where a lactam ring is formed between a side chain of lysine and a side chain of glutamic acid, but the ring is not particularly limited thereto. Examples of the peptide prepared by a combination of these methods may include peptides, in which the amino acid sequences thereof differ from that of native glucagon in at least one amino acid, and

the α-carbon in the N-terminus thereof is removed, while having activities to a glucagon receptor, a GLP-1 receptor, and a GIP receptor, *etc.,* but the peptide is not limited thereto, and the peptide may be prepared by a combination of various methods for the preparation of analogs.

Additionally, some of the amino acids may be substituted with other amino acids or non-natural compounds to avoid recognition by a degradation enzyme for increasing the *in vivo* half-life of the peptide, but the peptide is not particularly limited thereto.

Specifically, the peptide may be one in which the *in vivo* half-life is increased by avoiding recognition by the degradation enzyme through a substitution of the $2^{nd}$ amino acid sequence among the amino acid sequences of the peptide, but any substitution or modification of amino acids to avoid recognition by an *in vivo* degradation enzyme is included without limitation.

Additionally, such a modification for preparing a peptide includes all of the modifications using L-type or D-type amino acids and/or non-natural amino acids; and/or a modification of native sequence, for example, a modification of a side chain functional group, an intramolecular covalent bonding (*e.g.*, ring formation between side chains), methylation, acylation, ubiquitination, phosphorylation, aminohexanation, biotinylation, *etc.*

Additionally, the modification also includes all of those where one or more amino acids are added to the amino and/or carboxy terminus of native glucagon.

As the amino acids for the substitution or addition, not only the 20 amino acids commonly found in human proteins, but also atypical or non-naturally occurring amino acids may be used. Commercial sources of atypical amino acids include Sigma-Aldrich, ChemPep Inc., and Genzyme Pharmaceuticals. The peptides, where these amino acids are included, and typical peptide sequences may be synthesized and purchased from commercial peptide synthesis companies (*e.g*., American Peptide Company, Bachem (USA), or Anygen (Korea)).

Amino acid derivatives may be obtained in the same manner, and as one such example, 4-imidazoacetic acid, *etc.* may be used.

Additionally, the peptide may be in the form of a variant where the N-terminus and/or C-terminus, *etc.* of the peptide is chemically modified or protected by organic groups, or amino acids may be added to the terminus of the peptide, for its protection from proteases *in vivo* while increasing its stability.

In particular, in the case of a chemically synthesized peptide, its N- and C-termini are electrically charged. Therefore, in order to remove such charge, the N-terminus of the peptide may be acetylated and/or the C-terminus of the peptide may be amidated, but the peptide modification is not particularly limited thereto.

Additionally, the peptide includes all of those in the form of the peptide itself, a salt thereof (*e.g.,* a pharmaceutically acceptable salt thereof), or a solvate thereof. Additionally, the peptide may be in any pharmaceutically acceptable form.

The kind of the salt is not particularly limited. However, the salt is preferably one that is in a safe and effective form for a subject (*e.g.,* a mammal), but the salt is not particularly limited thereto.

The term "pharmaceutically acceptable" refers to a material which can be effectively used for a desired use within the scope of pharmaco-medical decision without inducing excessive toxicity, irritation, allergic responses, *etc.*

As used herein, the term "pharmaceutically acceptable salt" refers to a salt derived from pharmaceutically acceptable inorganic acids, organic acids, or bases. Examples of the suitable acids may include hydrochloric acid, bromic acid, sulfuric acid, nitric acid, perchloric acid, fumaric acid, maleic acid, phosphoric acid, glycolic acid, lactic acid, salicylic acid, succinic acid, toluene-*p*-sulfonic acid, tartaric acid, acetic acid, citric acid, methanesulfonic acid, formic acid, benzoic acid, malonic acid, naphthalene-2-sulfonic acid, benzenesulfonic acid, *etc.* The salts derived from suitable bases may include alkali metals (*e.g.,* sodium, potassium, *etc.*); alkali earth metals (*e.g.,* magnesium); ammonium, *etc.*

As used herein, the term "solvate" refers to a complex formed between the peptide or salt thereof and a solvent molecule.

The peptide of the disclosure (not claimed) may be a peptide which includes an amino acid sequence represented by General Formula 1 below:

Xaa1-Xaa2-Xaa3-Gly-Thr-Phe-Xaa7-Ser-Asp-Xaa10-Ser-Xaa12-Xaa13-Xaa14-Xaa15-Xaa16-Xaa17-Xaa18-Xaa19-Xaa20-Xaa21-Phe-Xaa23-Xaa24-Trp-Leu-Xaa27-Xaa28-Xaa29-Xaa30-R1 (General Formula 1, SEQ ID NO: 103).

In General Formula 1 above,

Xaa1 is histidine, 4-imidazoacetyl, or tyrosine;
Xaa2 is glycine, α-methyl-glutamic acid, or Aib;
Xaa3 is glutamic acid or glutamine;

Xaa7 is threonine or isoleucine;
Xaa10 is leucine, tyrosine, lysine, cysteine, or valine;
Xaa12 is lysine, serine, or isoleucine;
Xaa13 is glutamine, tyrosine, alanine, or cysteine;
Xaa14 is leucine, methionine, or tyrosine;
Xaa15 is cysteine, aspartic acid, glutamic acid, or leucine;
Xaa16 is glycine, glutamic acid, or serine;
Xaa17 is glutamine, arginine, isoleucine, glutamic acid, cysteine, or lysine;
Xaa18 is alanine, glutamine, arginine, or histidine;
Xaa19 is alanine, glutamine, cysteine, or valine;
Xaa20 is lysine, glutamine, or arginine;
Xaa21 is glutamic acid, glutamine, leucine, cysteine, or aspartic acid;
Xaa23 is isoleucine or valine;
Xaa24 is alanine, glutamine, cysteine, asparagine, aspartic acid, or glutamic acid;
Xaa27 is valine, leucine, or lysine;
Xaa28 is cysteine, lysine, alanine, asparagine, or aspartic acid;
Xaa29 is cysteine, glycine, glutamine, threonine, glutamic acid, or histidine;
Xaa30 is cysteine, glycine, lysine, or histidine, or is absent; and
R1 is cysteine, GKKNDWKHNIT (SEQ ID NO: 106), m-SSGAPPPS-n (SEQ ID NO: 107), or m-SSGQPPPS-n (SEQ ID NO: 108), or is absent, wherein:

m is -Cys-, -Pro-, or -Gly-Pro-; and
n is -Cys-, -Gly-, -Ser-, or -His-Gly-, or is absent.

Examples of the triple agonist of the disclosure may be those which include an amino acid sequence selected from the group consisting of SEQ ID NOS: 1 to 11 and SEQ ID NOS: 13 to 102; and those which (essentially) consist of an amino acid sequence selected from the group consisting of SEQ ID NOS: 1 to 11 and SEQ ID NOS: 13 to 102, but the triple agonist is not limited thereto.

Additionally, although described as "a peptide consisting of a particular SEQ ID NO" such description does not exclude a mutation that may occur by the addition of a meaningless sequence upstream or downstream of the amino acid sequence of the corresponding SEQ ID NO, or a mutation that may occur naturally, or a silent mutation thereof, as long as the peptide has an activity identical or corresponding to that of the peptide which consists of the amino acid sequence of the corresponding SEQ ID NO even when the peptide has such a sequence addition or mutation therein.

The above may be applicable to other specific cases of the present disclosure, but is not limited thereto.

Specifically, in General Formula 1 above, Xaa14 may be leucine or methionine, and Xaa15 may be cysteine, aspartic acid, or leucine.

Examples of such a peptide may include a peptide which includes an amino acid sequence selected from the group consisting of SEQ ID NOS: 1 to 11, 14 to 17, and 21 to 102; or a peptide which (essentially) consists of the same, but the peptide is not particularly limited thereto.

The peptide may significantly activate at least one of a glucagon receptor, a GLP-1 receptor, and a GIP receptor, but the peptide is not particularly limited thereto. Specifically, the peptide may be one which significantly activates a GLP-1 receptor, or additionally, significantly activates a glucagon receptor and/or a GIP receptor, but the peptide is not particularly limited thereto.

More specifically, the peptide may be one,
wherein in General Formula 1 above,

Xaa2 is glycine, $\alpha$-methyl-glutamic acid, or Aib;
Xaa7 is threonine;
Xaa10 is tyrosine, cysteine, or valine;
Xaa12 is lysine or isoleucine;
Xaa13 is tyrosine, alanine, glutamine, or cysteine;
Xaa14 is leucine, cysteine, or methionine;
Xaa15 is cysteine, leucine, glutamic acid, or aspartic acid;
Xaa17 is glutamine, arginine, isoleucine, cysteine, glutamic acid, or lysine;
Xaa18 is alanine, glutamine, arginine, or histidine;
Xaa19 is alanine, glutamine, valine, or cysteine;
Xaa20 is lysine, arginine, or glutamine;
Xaa21 is glutamic acid, glutamine, leucine, cysteine, or aspartic acid;

Xaa23 is isoleucine or valine;
Xaa24 is cysteine, alanine, glutamine, asparagine, glutamic acid, or aspartic acid; and
Xaa27 is leucine or lysine,
but the peptide is not particularly limited thereto.

More specifically, the peptide may be one, wherein in General Formula 1 above,

Xaa2 is glycine, α-methyl-glutamic acid, or Aib;
Xaa7 is threonine;
Xaa10 is tyrosine, cysteine, or valine;
Xaa12 is lysine or isoleucine;
Xaa13 is tyrosine, alanine, or cysteine;
Xaa14 is leucine or methionine;
Xaa15 is cysteine or aspartic acid;
Xaa17 is glutamine, arginine, isoleucine, cysteine, or lysine;
Xaa18 is alanine, arginine, or histidine;
Xaa19 is alanine, glutamine, or cysteine;
Xaa20 is lysine or glutamine;
Xaa21 is glutamic acid, cysteine, or aspartic acid;
Xaa23 is valine;
Xaa24 is alanine, glutamine, cysteine, asparagine, or aspartic acid; and
Xaa27 is leucine or lysine, but the peptide is not particularly limited thereto.

More specifically, the peptide may be one, wherein in General Formula 1 above,

Xaa2 is α-methyl-glutamic acid or Aib;
Xaa7 is threonine;
Xaa10 is tyrosine or cysteine;
Xaa12 is lysine or isoleucine;
Xaa13 is tyrosine, alanine, or cysteine;
Xaa14 is leucine or methionine;
Xaa15 is cysteine or aspartic acid;
Xaa16 is glutamic acid;
Xaa17 is arginine, isoleucine, cysteine, or lysine;
Xaa18 is alanine, arginine, or histidine;
Xaa19 is alanine, glutamine, or cysteine;
Xaa20 is lysine or glutamine;
Xaa21 is glutamic acid or aspartic acid;
Xaa23 is valine;
Xaa24 is glutamine, asparagine, or aspartic acid;
Xaa27 is leucine; and
Xaa28 is cysteine, alanine, asparagine, or aspartic acid.

Specifically, the peptide may be one, wherein in General Formula 1 above,

Xaa1 is histidine or 4-imidazoacetyl;
Xaa2 is α-methyl-glutamic acid or Aib;
Xaa3 is glutamine;
Xaa7 is threonine;
Xaa10 is tyrosine;
Xaa12 is isoleucine;
Xaa13 is alanine or cysteine;
Xaa14 is methionine;
Xaa15 is aspartic acid;
Xaa16 is glutamic acid;

Xaa17 is isoleucine or lysine;
Xaa18 is alanine or histidine;
Xaa19 is glutamine or cysteine;
Xaa20 is lysine;
Xaa21 is aspartic acid;
Xaa23 is valine;
Xaa24 is asparagine;
Xaa27 is leucine;
Xaa28 is alanine or asparagine;
Xaa29 is glutamine or threonine; and
Xaa30 is cysteine or lysine, or is absent.

More specifically, the peptide may be one, wherein in General Formula 1 above,

Xaa2 is glycine, α-methyl-glutamic acid, or Aib;
Xaa3 is glutamine;
Xaa7 is threonine;
Xaa10 is tyrosine, cysteine, or valine;
Xaa12 is lysine;
Xaa13 is tyrosine;
Xaa14 is leucine;
Xaa15 is aspartic acid;
Xaa16 is glycine, glutamic acid, or serine;
Xaa17 is glutamine, arginine, cysteine, or lysine;
Xaa18 is alanine, arginine, or histidine;
Xaa19 is alanine or glutamine;
Xaa20 is lysine or glutamine;
Xaa21 is glutamic acid, cysteine, or aspartic acid;
Xaa23 is valine;
Xaa24 is alanine, glutamine, or cysteine;
Xaa27 is leucine or lysine; and
Xaa29 is glycine, glutamine, threonine, or histidine, but the peptide is not particularly limited thereto.

Such a peptide may correspond to a case where the peptide has significant activation levels on both the GLP-1 receptor and glucagon receptor, or higher activation levels compared to that on the GIP receptor; a case where the peptide has significant activation levels on all of the GLP-1 receptor, glucagon receptor, and GIP receptor; or a case where the peptide has significant activation levels on both the GLP-1 receptor and GIP receptor and higher activation levels compared to that on the glucagon receptor; but the cases are not particularly limited thereto.

Examples of the peptide of the disclosure may include a peptide which includes an amino acid sequence selected from the group consisting of SEQ ID NOS: 8, 9, 21 to 37, 39, 42, 43, 49 to 61, 64 to 83, 85, 86, 88, 89, 91 to 93, and 95 to 102; or a peptide which (essentially) consists of the same, but the peptide is not particularly limited thereto.

The peptide of the disclosure may include an amino acid sequence represented by General Formula 2 below.

Xaa1-Xaa2-Gln-Gly-Thr-Phe-Thr-Ser-Asp-Xaa10-Ser-Lys-Xaa13-Xaa14-Xaa15-Xaa16-Xaa17-Xaa18-Xaa19-Xaa20-Xaa21-Phe-Xaa23-Xaa24-Trp-Leu-Leu-Xaa28-Xaa29-Xaa30-Xaa31-Ser-Ser-Gly-Gln-Pro-Pro-Pro-Ser-Xaa40 (General Formula 2, SEQ ID NO: 104)

In General Formula 2 above, the peptide may be one where:

Xaa1 is 4-imidazoacetyl, histidine, or tyrosine;
Xaa2 is glycine, α-methyl-glutamic acid, or Aib;
Xaa10 is tyrosine or cysteine;
Xaa13 is alanine, glutamine, tyrosine, or cysteine;
Xaa14 is leucine, methionine, or tyrosine;
Xaa15 is aspartic acid, glutamic acid, or leucine;

Xaa16 is glycine, glutamic acid, or serine;
Xaa17 is glutamine, arginine, isoleucine, glutamic acid, cysteine, or lysine;
Xaa18 is alanine, glutamine, arginine, or histidine;
Xaa19 is alanine, glutamine, cysteine, or valine;
Xaa20 is lysine, glutamine, or arginine;
Xaa21 is cysteine, glutamic acid, glutamine, leucine, or aspartic acid;
Xaa23 is isoleucine or valine;
Xaa24 is cysteine, alanine, glutamine, asparagine, or glutamic acid;
Xaa28 is lysine, cysteine, asparagine, or aspartic acid;
Xaa29 is glycine, glutamine, cysteine, or histidine;
Xaa30 is cysteine, glycine, lysine, or histidine;
Xaa31 is proline or cysteine; and
Xaa40 is cysteine or is absent.

More specifically, the peptide may be one, where in General Formula 2,

Xaa13 is alanine, tyrosine, or cysteine;
Xaa15 is aspartic acid or glutamic acid,
Xaa17 is glutamine, arginine, cysteine, or lysine;
Xaa18 is alanine, arginine, or histidine;
Xaa21 is cysteine, glutamic acid, glutamine, or aspartic acid;
Xaa23 is isoleucine or valine;
Xaa24 is cysteine, glutamine, or asparagine,
Xaa28 is cysteine, asparagine, or aspartic acid;
Xaa29 is glutamine, cysteine, or histidine; and
Xaa30 is cysteine, lysine, or histidine.

Examples of the peptide of the disclosure may include a peptide which includes an amino acid sequence selected from the group consisting of SEQ ID NOS: 21, 22, 42, 43, 50, 64 to 77, and 95 to 102; more specifically, a peptide which includes an amino acid sequence selected from the group consisting of SEQ ID NOS: 21, 22, 42, 43, 50, 64 to 77, and 96 to 102; or a peptide which (essentially) consists of the same, but the peptide is not particularly limited thereto.

The peptide of the disclosure may include an amino acid sequence represented by General Formula 3 below.

Xaa1-Xaa2-Gln-Gly-Thr-Phe-Thr-Ser-Asp-Tyr-Ser-Lys-Xaa13-Leu-Asp-Glu-Xaa17-Xaa18-Xaa19-Lys-Xaa21-Phe-Val-Xaa24-Trp-Leu-Leu-Xaa28-Xaa29-Xaa30-Xaa31-Ser-Ser-Gly-Gln-Pro-Pro-Pro-Ser-Xaa40 (General Formula 3, SEQ ID NO: 105).

The peptide may be one, where in General Formula 3 above,

Xaa1 is histidine or tyrosine;
Xaa2 is α-methyl-glutamic acid or Aib;
Xaa13 is alanine, tyrosine or cysteine;
Xaa17 is arginine, cysteine, or lysine;
Xaa18 is alanine or arginine;
Xaa19 is alanine or cysteine;
Xaa21 is glutamic acid or aspartic acid;
Xaa24 is glutamine or asparagine;
Xaa28 is cysteine or aspartic acid;
Xaa29 is cysteine, histidine, or glutamine;
Xaa30 is cysteine or histidine;
Xaa31 is proline or cysteine; and
Xaa40 is cysteine or is absent.

Examples of the peptide of the disclosure may include a peptide which includes an amino acid sequence selected from the group consisting of SEQ ID NOS: 21, 22, 42, 43, 50, 64 to 71, 75 to 77, and 96 to 102; or a peptide

which (essentially) consists of the same, but the peptide is not particularly limited thereto.

Additionally, the peptide may be one, wherein in General Formula 1 above, R1 is cysteine, GKKNDWKHNIT (SEQ ID NO: 106), CSSGQPPPS (SEQ ID NO: 109), GPSSGAPPPS (SEQ ID NO: 110), GPSSGAPPPSC (SEQ ID NO: 111), PSSGAPPPS (SEQ ID NO: 112), PSSGAPPPSG (SEQ ID NO: 113), PSSGAPPPSHG (SEQ ID NO: 114), PSSGAPPPSS (SEQ ID NO: 115), PSSGQPPPS (SEQ ID NO: 116), or PSSGQPPPSC (SEQ ID NO: 117), or is absent, but the peptide is not particularly limited thereto.

Additionally, the peptide may be synthesized according to its length by a method well known in the art (e.g., by an automatic peptide synthesizer) and may be produced by genetic engineering technology.

Specifically, the peptide may be prepared by a standard synthesis method, a recombinant expression system, or any other method known in the art. Accordingly, the peptide may be synthesized by many methods including, for example, the methods described below:

(a) a method of synthesizing a peptide by a solid-phase or liquid-phase method stepwise or by fragment assembly, followed by isolation and purification of the final peptide product; or
(b) a method of expressing a nucleic acid construct encoding a peptide in a host cell and recovering the expression product from the host cell culture; or
(c) a method of performing an *in vitro* cell-free expression of a nucleic acid construct encoding a peptide and recovering the expression product therefrom; or

a method of obtaining fragments of a peptide by any combination of the methods (a), (b), and (c), obtaining the peptide by linking the peptide fragments, and then recovering the peptide.

Specifically, the composition is a pharmaceutical composition for the prevention or treatment of liver disease, and it may be a pharmaceutical composition that contains a pharmaceutically acceptable excipient; and a peptide comprising an amino acid sequence of any one of SEQ ID NOS: 1 to 102 (not claimed) or a peptide consisting (essentially) thereof in a pharmaceutically effective amount.

The peptide of the disclosure may be one which includes an amino acid sequence selected from the group consisting of SEQ ID NOS: 21, 22, 42, 43, 50, 64, 66, 67, 70, 71, 76, 77, 96, 97, and 100, or (essentially) consists of the same; one which includes an amino acid sequence selected from the group consisting of SEQ ID NOS: 21, 22, 42, 43, 50, 66, 67, 77, 96, 97, and 100, or (essentially) consists of the same; or one which includes an amino acid sequence selected from the group consisting of SEQ ID NOS: 21, 22, 42, 43, 50, 77, and 96, or (essentially) consists of the same; but the peptide is not limited thereto. Additionally, the peptide is in the form of a long-acting conjugate, and the long-acting conjugate may be one in which a biocompatible material for increasing the *in vivo* half-life of a peptide is linked to a peptide having activities to a glucagon receptor, a GLP-1 receptor, and a GIP receptor. In the present specification, the biocompatible material can be used interchangeably with a carrier.

In the present disclosure, a conjugate of the peptide can exhibit an enhanced duration of efficacy compared to the peptide to which a carrier is not linked, and in the present disclosure, such a conjugate is referred to as a "long-acting conjugate", which may be used interchangeably with "conjugate".

Meanwhile, the conjugate may be one that is not naturally occurring.

Specifically, the long-acting conjugate may be one that is represented by Formula 1 below, but the long-acting conjugate is not limited thereto.

[Formula 1] X-L-F

wherein in Formula 1 above,

X is a peptide of SEQ ID NO:42
L is a linker containing an ethylene glycol repeat unit;
F is an immunoglobulin Fc fragment or a derivative thereof; and
'-' represents a covalent bond between X and L and between L and F.

In the above conjugate, F is a material capable of increasing the half-life of X (*i.e.,* a peptide having activities to a glucagon receptor, a GLP-1 receptor, and a GIP receptor; and specifically, a peptide containing an amino acid sequence of any one of SEQ ID NOS: 1 to 102) and it corresponds to a constitution of the moiety that constitutes the conjugate of the present disclosure.

The F may be one which is linked to X by a covalent chemical bond or non-covalent chemical bond, and specifically, the F and the X may be linked to each other through L by a covalent chemical bond.

The F may be an immunoglobulin Fc fragment or a derivative thereof, and more specifically, the immunoglobulin Fc fragment or its derivative may be derived from IgG, but the F is not particularly limited thereto.

In the present disclosure, the term "immunoglobulin Fc fragment" refers to a region which includes the heavy chain constant region 2 (CH2) and/or heavy chain constant region 3 (CH3) portions, excluding the variable regions of immunoglobulin heavy and light chains. The immunoglobulin Fc fragment may be a constitution constituting the moiety of the conjugate of the present disclosure.

In the present disclosure, an Fc fragment includes not only the native sequence obtained by papain digestion of immunoglobulin, but also a derivative thereof (e.g., sequences in which one or more amino acid residues in the native sequence are modified by deletion, insertion, non-conservative or conservative substitution, or a combination thereof), and is thus different from that of the native form.

The F may have a structure in which two polypeptide chains are linked by a disulfide bond, or a structure in which two polypeptide chains are linked through a nitrogen atom in only one of the two chains, but the structure of the F is not limited thereto. The linkage through the nitrogen atom may be linked to the epsilon N atom or the N-terminus amino group of lysine via reductive amination.

The reductive amination reaction refers to a reaction in which an amine group or amino group of a reactant reacts with an aldehyde of another reactant (*i.e.*, a functional group capable of reductive amination) to produce an amine, and an amine bond is formed by a reduction reaction thereafter. The reductive amination reaction is a reaction of organic synthesis widely known in the art.

The F may be one which is linked through a nitrogen atom of the N-terminus proline, but the F is not limited thereto.

Such an immunoglobulin Fc fragment may include a hinge region in the heavy chain constant region, but is not limited thereto.

In the present disclosure, the immunoglobulin Fc fragment may include a specific hinge sequence in the N-terminus.

As used herein, the term "hinge sequence" refers to a region which is located in the heavy chain and forms a dimer of immunoglobulin Fc fragments through an inter-disulfide bond.

In the present disclosure, the hinge sequence may be a modified sequence in which part of the hinge sequence having the following amino acid sequence is deleted and thus there is only one cysteine residue in the sequence, but the hinge sequence is not limited thereto:

Glu-Ser-Lys-Tyr-Gly-Pro-Pro-Cys-Pro-Ser-Cys-Pro (SEQ ID NO: 119).

The hinge sequence may be one in which the $8^{th}$ or $11^{th}$ cysteine residue in the hinge sequence of SEQ ID NO: 119 is deleted and thus only one cysteine residue is included in the sequence. The hinge sequence of the present disclosure may consist of 3 to 12 amino acids, including only one cysteine residue, but the hinge sequence is not limited thereto. More specifically, the hinge sequence may have the following sequences: Glu-Ser-Lys-Tyr-Gly-Pro-Pro-Pro-Ser-Cys-Pro (SEQ ID NO: 120), Glu-Ser-Lys-Tyr-Gly-Pro-Pro-Cys-Pro-Ser-Pro (SEQ ID NO: 121), Glu-Ser-Lys-Tyr-Gly-Pro-Pro-Cys-Pro-Ser (SEQ ID NO: 122), Glu-Ser-Lys-Tyr-Gly-Pro-Pro-Cys-Pro-Pro (SEQ ID NO: 123), Lys-Tyr-Gly-Pro-Pro-Cys-Pro-Ser (SEQ ID NO: 124), Glu-Ser-Lys-Tyr-Gly-Pro-Pro-Cys (SEQ ID NO: 125), Glu-Lys-Tyr-Gly-Pro-Pro-Cys (SEQ ID NO: 126), Glu-Ser-Pro-Ser-Cys-Pro (SEQ ID NO: 127), Glu-Pro-Ser-Cys-Pro (SEQ ID NO: 128), Pro-Ser-Cys-Pro (SEQ ID NO: 129), Glu-Ser-Lys-Tyr-Gly-Pro-Pro-Ser-Cys-Pro (SEQ ID NO: 130), Lys-Tyr-Gly-Pro-Pro-Pro-Ser-Cys-Pro (SEQ ID NO: 131), Glu-Ser-Lys-Tyr-Gly-Pro-Ser-Cys-Pro (SEQ ID NO: 132), Glu-Ser-Lys-Tyr-Gly-Pro-Pro-Cys (SEQ ID NO: 133), Lys-Tyr-Gly-Pro-Pro-Cys-Pro (SEQ ID NO: 134), Glu-Ser-Lys-Pro-Ser-Cys-Pro (SEQ ID NO: 135), Glu-Ser-Pro-Ser-Cys-Pro (SEQ ID NO: 136), Glu-Pro-Ser-Cys (SEQ ID NO: 137), and Ser-Cys-Pro (SEQ ID NO: 138).

More specifically, the hinge sequence may be one which includes the amino acid sequence of SEQ ID NO: 129 (Pro-Ser-Cys-Pro) or SEQ ID NO: 138 (Ser-Cys-Pro), but the hinge sequence is not limited thereto.

The immunoglobulin Fc fragment may be in the form in which two molecules of the immunoglobulin Fc chain form a dimer due to the presence of a hinge sequence therein, and in addition, the conjugate of Formula 1 may be in the form in which one end of the linker is linked to one chain of the dimeric immunoglobulin Fc fragments, but the immunoglobulin Fc fragment and the conjugate of Formula 1 are not limited thereto.

As used herein, the term "N-terminus" refers to the amino terminus of a protein or polypeptide, and it may include 1, 2, 3, 4, 5, 6, 7, 8, 9, or 10 or more amino acids from the most terminal end or the terminal end of the amino terminus. The immunoglobulin Fc fragment of may include a hinge sequence in the N-terminus, but the immunoglobulin Fc fragment is not limited thereto.

In addition, the immunoglobulin Fc fragment may be an extended Fc fragment, which includes all or part of the heavy chain constant region 1 (CH1) and/or light chain constant region 1 (CL1) excluding the heavy chain and light chain variable regions of an immunoglobulin, as long as it has substantially the same or an improved effect compared to its native type. In addition, the immunoglobulin Fc fragment may be a fragment in which some fairly long amino acid sequences corresponding to CH2 and/or CH3 are removed.

For example, the immunoglobulin Fc fragment may be 1) a CH1 domain, a CH2 domain, a CH3 domain, and a CH4 domain; 2) a CH1 domain and a CH2 domain; 3) a CH1 domain and a CH3 domain; 4) a CH2 domain and a CH3 domain; 5) a combination between one or two or more domains among a CH1 domain, a CH2 domain, a CH3 domain,

and a CH4 domain and an immunoglobulin hinge region (or part of the hinge region); and 6) a dimer of each domain of the heavy chain constant region and a light chain constant region, but the immunoglobulin Fc fragment is not limited thereto.

In addition, the immunoglobulin Fc fragment may be in a dimeric form, or one molecule of X may be covalently linked to one Fc fragment in a dimeric form, and in particular, the immunoglobulin Fc and X may be linked to each other by a non-peptide polymer.

Meanwhile, it is also possible that two molecules of X are symmetrically bound to one Fc fragment in a dimeric form. In particular, the immunoglobulin Fc and X may be linked to each other by a non-peptide linker, but the linkage between the immunoglobulin Fc fragment and X is not limited to the examples described above.

In addition, the immunoglobulin Fc fragment includes natural amino acid sequences as well as sequence derivatives thereof. The amino acid sequence derivative means that the sequence of the amino acid is different from that of its natural amino acid due to the presence of deletion, addition, conservative or non-conservative substitution, or a combination thereof in one or more amino acid residues in the sequence of the natural amino acid.

For example, amino acid residues at positions 214 to 238, 297 to 299, 318 to 322, or 327 to 331 in IgG Fc, which are known to be important for linkage, may be used as the sites suitable for modification.

Additionally, various types of derivatives are possible, for example, one where the site capable of forming an inter-disulfide bond is removed; one where several N-terminal amino acids from native Fc are removed; one where a methionine residue is added to the N-terminus of native Fc, *etc.* Additionally, complement binding sites (*e.g.*, C1q binding sites) or antibody-dependent cell-mediated cytotoxicity (ADCC) sites may be removed to remove the effector function. The techniques for preparing the sequence derivatives of an immunoglobulin Fc fragment are disclosed in International Publication Nos. WO 97/34631, WO 96/32478, *etc.*

Amino acid substitutions in a protein or peptide that do not alter the entire activity of a molecule are well known in the art (H. Neurath, R. L. Hill, The Proteins, Academic Press, New York, 1979). The most common substitutions occur between amino acid residues of Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, amino acids may be modified by phosphorylation, sulfation, acrylation, glycosylation, methylation, famesylation, acetylation, amidation, *etc.*

Additionally, the Fc derivatives described above may be those which exhibit the same biological activity as the Fc region and have increased structural stability of the Fc region against heat, pH, *etc.*

Additionally, such an Fc fragment may be obtained from a native type isolated from humans or animals (*e.g.*, cows, goats, pigs, mice, rabbits, hamsters, rats, guinea pigs, *etc.*) or may be recombinants or derivatives thereof obtained from transformed animal cells or microorganisms. In particular, the Fc fragment may be obtained from native Fc by isolating whole immunoglobulins from human or animal organisms and treating them with a protease. Papain treatment of the Fc fragment generates Fab and Fc fragments, and pepsin treatment of the Fc fragment produces pF'c and $F(ab)_2$ fragments. These fragments may be subjected to size exclusion chromatography to isolate Fc or pF'c. The Fc fragment may be a recombinant immunoglobulin Fc fragment where a human-derived Fc fragment is obtained from a microorganism.

Additionally, the immunoglobulin Fc fragment may be in the form of native glycan, increased glycans compared to its native type, decreased glycans compared to its native type, or in a deglycosylated form. The increase, decrease, or removal of the immunoglobulin Fc glycans may be achieved by conventional methods such as a chemical method, enzymatic method, and genetic engineering method using a microorganism. In particular, the immunoglobulin Fc fragment where the glycans are removed from the Fc shows a significant decrease in binding affinity for the complement (C1q) and a decrease or removal of antibody-dependent cytotoxicity or complement-dependent cytotoxicity, and thus it does not induce unnecessary immune responses *in vivo.* In this regard, an immunoglobulin Fc fragment in a deglycosylated or aglycosylated form may be more suitable to meet the original object of the present disclosure as a drug carrier.

As used herein, the term "deglycosylation" means removal of glycans from an Fc fragment with an enzyme, and the term "aglycosylation" means that an Fc fragment is produced in an unglycosylated form in prokaryotes, and in a more specific example, in *E. coli.*

Meanwhile, the immunoglobulin Fc fragment may be derived from humans or animals (*e.g.*, cows, goats, pigs, mice, rabbits, hamsters, rats, guinea pigs, *etc.*), and in a more specific example, it may be derived from humans.

Additionally, the immunoglobulin Fc fragment may be derived from IgG, IgA, IgD, IgE, IgM, or a combination or hybrid thereof. The immunoglobulin Fc fragment may be derived from IgG or IgM, which are among the most abundant proteins in human blood, and it may be derived from IgG, which is known to enhance the half-lives of ligand-binding proteins. The immunoglobulin Fc fragment may be an IgG4 Fc fragment, and it may be an aglycosylated Fc fragment derived from a human IgG4, but the immunoglobulin Fc fragment is not limited thereto.

Additionally, the immunoglobulin Fc fragment, being a human IgG4 fragment, may be in the form of a homodimer in which two monomers are linked through an inter-disulfide bond (an inter-chain form) between cysteines, which are the $3^{rd}$ amino acid of each monomer. In particular, each monomer of the homodimer independently has/or can have an internal disulfide bond between the cysteines at positions 35 and 95; and an internal disulfide bond between the cysteines

at positions 141 and 199 (*i.e.,* two internal disulfide bonds (an intra-chain form)). With respect to the number of amino acids, each monomer may consist of 221 amino acids, and the amino acids forming the homodimer may consist of a total of 442 amino acids, but the number of amino acids is not limited thereto.

Specifically, the immunoglobulin Fc fragment may be one in which two monomers having the amino acid sequence of SEQ ID NO: 139 (consisting of 221 amino acids) form a homodimer through an inter-disulfide bond between cysteines, which are the 3rd amino acid of each monomer, and in which the monomers of the homodimer independently form an internal disulfide bond between the cysteines at positions 35 and 95 and an internal disulfide bond between the cysteines at positions 141 and 199, but the immunoglobulin Fc fragment is not limited thereto.

As used herein, the term "combination" means that polypeptides encoding single-chain immunoglobulin Fc fragments of the same origin are linked to a single-chain polypeptide of a different origin to form a dimer or multimer. That is, it is possible to prepare a dimer or multimer from two or more fragments selected from the group consisting of Fc fragments of IgG Fc, IgA Fc, IgM Fc, IgD Fc, and IgE Fc.

Meanwhile, the L may be a non-peptide linker, for example, a linker containing an ethylene glycol repeat unit.

In the present disclosure, the term "non-peptide linker" includes a biocompatible polymer in which two or more repeat units are linked. The repeat units are linked to each other through any covalent bond which is not a peptide bond. The non-peptide linker may be one constitution that constitutes the moieties of the conjugate, and it corresponds to L in Formula 1 above. As the non-peptide linker that can be used in the present disclosure, any polymer which has a resistance to proteases *in vivo* can be used without limitation. In the present disclosure, the non-peptide linker can be used interchangeably with a non-peptide polymer.

Although not particularly limited, the non-peptide linker may be a linker containing an ethylene glycol repeat unit (*e.g.*, polyethylene glycol), and additionally, those derivatives which are already known in the art and the derivatives that can easily be prepared at the technological level of those skilled in the art are included in the scope of the present disclosure.

The repeat unit of the non-peptide linker may be an ethylene glycol repeat unit, and specifically, the non-peptide linker may be one which includes a functional group used for the preparation of the conjugate at an end while including an ethylene glycol repeat unit. The long-acting conjugate may be in the form in which X and F are linked through the functional group, but the long-acting conjugate is not limited thereto. The non-peptide linker may include two, or three or more functional groups, and each functional group may be the same as or different from each other, but the non-peptide linker is not limited thereto.

Specifically, the linker may be polyethylene glycol (PEG) represented by Formula 2 below, but the linker is not limited thereto:

[Formula 2]

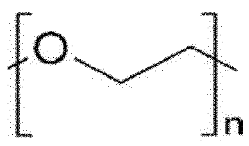

wherein n is 10 to 2,400, n is 10 to 480, or n is 50 to 250, but the range of n is not limited thereto.

In the long-acting conjugate above, the PEG moiety may include not only the $-(CH_2CH_2O)_n-$ structure, but also an oxygen atom interposed between a linking element and the $-(CH_2CH_2O)_n-$ structure, but the PEG moiety is not limited thereto.

Additionally, the conjugate may have a structure in which an immunoglobulin fragment (F) is linked to a peptide (X) containing an amino acid sequence of any one of SEQ ID NOS: 1 to 102 by a covalent bond through a linker containing an ethylene glycol repeat unit, but the structure of the conjugate is not limited thereto. The polyethylene glycol is a general term including all of the forms of homopolymers of ethylene glycol, PEG copolymers, and monomethyl-substituted PEG polymers (mPEG), but the polyethylene glycol is not particularly limited thereto.

The molecular weight of the non-peptide polymer may be in the range of 1 kDa to 100 kDa, specifically 1 kDa to 20 kDa, or 1 kDa to 10 kDa, but the molecular weight of the non-peptide polymer is not limited thereto. Additionally, the non-peptide linker, which is linked to the polypeptide corresponding to the F, may include not only a single kind of a polymer but also a combination of different kinds of polymers.

In a specific example, one end of the non-peptide linker can be linked to an amine group or thiol group of F (*e.g.*, an immunoglobulin Fc fragment) while the other end can be linked to an amine group or thiol group of X.

Specifically, the non-peptide polymer may include a reactive group which can be linked to F (*e.g.*, an immunoglobulin Fc fragment) and X at both ends thereof, respectively, and more specifically, a reactive group which can be linked to an amine group located at the N-terminus or lysine, or a thiol group of cysteine of X; or an amine group located

at the N-terminus or lysine, or a thiol group of cysteine of F, but the non-peptide polymer is not limited thereto.

Additionally, the reactive group of the non-peptide polymer that can be linked to F (*e.g.*, an immunoglobulin Fc fragment) and X may be selected from the group consisting of an aldehyde group, a maleimide group, and a succinimide derivative, but the reactive group is not limited thereto.

In the above, as an example of the aldehyde group, a propionaldehyde group or butyraldehyde group may be used, but the aldehyde group is not limited thereto.

In the above, as a succinimide derivative, succinimidyl valerate, succinimidyl methylbutanoate, succinimidyl methylpropionate, succinimidyl butanoate, succinimidyl propionate, *N*-hydroxysuccinimide, hydroxy succinimidyl, succinimidyl carboxymethyl, or succinimidyl carbonate may be used, but the succinimide derivative is not limited thereto.

The non-peptide linker may be linked to X and F through these reactive groups, but the reactive groups are not particularly limited thereto.

Additionally, the final product produced through reductive amination by an aldehyde bond is much more stable than that linked by an amide bond. The aldehyde reactive group selectively reacts with a N-terminus at a low pH, while it can form a covalent bond with a lysine residue at a high pH (*e.g.,* pH 9.0).

Additionally, the reactive groups at each end of the non-peptide linker may be the same as or different from each other. For example, the non-peptide linker may have a maleimide reactive group at one end, while having an aldehyde group, a propionaldehyde group, or a butyraldehyde group at the other end. However, the reactive groups are not particularly limited thereto as long as F (specifically, an immunoglobulin Fc fragment) can be linked to X at each end of the non-peptide linker.

For example, the non-peptide linker may include, as a reactive group, a maleimide group at one end, while including an aldehyde group, a propionaldehyde group, or a butyraldehyde group at the other end.

When a polyethylene glycol having a reactive hydroxy group at both ends thereof is used as the non-peptide polymer, the long-acting protein conjugate may be prepared by activating the hydroxy group to various reactive groups by known chemical reactions or by using a commercially available polyethylene glycol having a modified reactive group.

In a specific example, the non-peptide polymer may be one which is linked to a cysteine residue of X, and more specifically, to the -SH group of cysteine, but the non-peptide polymer is not limited thereto.

For example, the non-peptide polymer may be one which is linked to a peptide corresponding to the X, at a position of the $10^{th}$ cysteine residue, the $13^{th}$ cysteine residue, the $15^{th}$ cysteine residue, the $17^{th}$ cysteine residue, the $19^{th}$ cysteine residue, the $21^{st}$ cysteine residue, the $24^{th}$ cysteine residue, the $28^{th}$ cysteine residue, the $29^{th}$ cysteine residue, the $30^{th}$ cysteine residue, the $31^{st}$ cysteine residue, the $40^{th}$ cysteine residue, or the $41^{st}$ cysteine residue, but the non-peptide polymer is not particularly limited thereto.

Specifically, a reactive group of the non-peptide polymer can be linked to the -SH group of the cysteine residue, and all of the descriptions above can apply to the reactive group. In a case where maleimide-PEG-aldehyde is used, the maleimide group is linked to the -SH group of X by a thioether bond, and the aldehyde group can be linked to F (specifically, a $-NH_2$ group of an immunoglobulin Fc) through a reductive amination reaction, but the linkage is not limited thereto.

In addition, in the conjugate above, the reactive group of the non-peptide polymer may be linked to $-NH_2$ located in the N-terminus of the immunoglobulin Fc fragment.

In addition, the conjugate may be one having an increased duration of efficacy compared to native GLP-1, GIP, or glucagon, or X, which is not modified by F, and such a conjugate includes not only the forms described above, but also all of the forms encapsulated in biodegradable nanoparticles, *etc.*

The peptide according to the present disclosure or a conjugate thereof may have a use of prevention or treatment of liver disease.

As used herein, the term "liver disease" refers to a disease occurring in the liver, and it may include metabolic liver disease or liver inflammation, but the liver disease is not limited thereto. Representative examples of the liver disease may include simple steatosis, non-alcoholic fatty liver (NAFL), liver inflammation, non-alcoholic steatohepatitis (NASH), cholestasis liver disease, liver fibrosis, cirrhosis, liver decompensation, liver cancer, *etc.,* and as long as an abnormality occurs in the tissues and functions of the liver, it may be the liver disease according to the present disclosure. In many cases, liver inflammation may occur due to causes of viruses, alcohol, drugs, immune disorders, metabolic diseases, *etc.,* and liver inflammation is known to develop into diseases such as cirrhosis, liver cancer, *etc.* according to the progression and chronicity of liver inflammation. The composition can show an effect on liver disease accompanied by or caused by liver inflammation (*e.g.*, liver inflammation, non-alcoholic steatohepatitis (NASH), or liver fibrosis), but the liver disease is not limited thereto.

Meanwhile, the composition can show an effect even for the prevention or treatment of the liver disease that does not accompany inflammation, and examples of such liver disease may include simple steatosis, non-alcoholic fatty liver (NAFL), cirrhosis, *etc.,* but the liver disease is not limited thereto.

The liver disease for which the peptide or a conjugate thereof has a therapeutic effect may be metabolic liver disease, but the liver disease is not limited thereto. The metabolic liver disease is a disease caused by an abnormal

chemical reaction of the body that interferes with the body's metabolism, and it includes simple steatosis, fatty liver, steatohepatitis, *etc.*

The composition may be one which shows a preventive or therapeutic effect on metabolic liver disease by reducing the amount of triglycerides and/or cholesterol in liver tissue when administered, but the composition is not limited thereto. The metabolic liver disease may or may not be accompanied by inflammation, and examples of liver diseases that can be treated with the composition may include simple steatosis, non-alcoholic fatty liver (NAFL), non-alcoholic steatohepatitis (NASH), *etc.,* but the liver diseases are not limited thereto.

The "nonalcoholic fatty liver disease (NAFLD)", which is a representative example of metabolic liver disease, refers to the case where this is accompanied by fatty liver even though the subject has no history of alcohol intake or is not related to alcohol intake. Fatty liver refers to the occurrence of a phenomenon in which triglycerides appear to be abnormally deposited in liver cells, unlike normal cases. About 5% of the normal liver is composed of adipose tissue. Although triglycerides, fatty acids, phospholipids, cholesterol, and cholesterol esters are the main components of fat, once fatty liver occurs, most of the components are replaced with triglycerides, and when the amount of triglycerides is 5% or higher relative to the liver weight, it is diagnosed as fatty liver. Fatty liver is caused by a disorder of fat metabolism in liver cells or a defect in the process of transporting excessive fat, *etc.* and it is mainly caused by a disorder of fat metabolism in the liver. Most of the fat accumulated in the fatty liver may be triglycerides.

The non-alcoholic steatohepatitis disease (NAFLD) refers to a group of diseases which includes simple steatosis with only excessive accumulation of fat in liver cells, non-alcoholic fatty liver (NAFL), non-alcoholic steatohepatitis (NASH) accompanied by hepatocellular necrosis, inflammation and fibrosis, *etc.,* but the non-alcoholic steatohepatitis disease (NAFLD) is not limited thereto as long as the disease can be treated with the composition. The non-alcoholic steatohepatitis disease (NAFLD) may be one which is accompanied by non-alcoholic steatohepatitis (NASH), but the non-alcoholic steatohepatitis disease (NAFLD) is not limited thereto.

In addition, the liver disease for which the peptide or a conjugate thereof has a therapeutic effect may be liver inflammation, but the liver disease is not limited thereto. As used herein, the term "liver inflammation", which is the most common cause of liver disease, refers to a disease that causes inflammation of the liver, and is divided into acute hepatitis and chronic hepatitis according to causes and symptoms. Viruses, alcohol, drugs, immune disorders, metabolic diseases, *etc.* are the main causes.

The composition according may reduce the expression of at least one of TNF-$\alpha$, MCP-1, and IL-6 in the liver tissue when administered, and through this, the composition may show a preventive or therapeutic effect on liver inflammation, but the effects of the composition are not limited thereto.

The peptide or a conjugate thereof can show not only the effect of alleviating the inflammation of the liver itself, but also a therapeutic effect on diseases accompanied by or caused by inflammation of the liver (*e.g.*, hepatitis, non-alcoholic steatohepatitis (NASH), liver fibrosis, *etc.*).

As used herein, "non-alcoholic steatohepatitis (NASH))", which is one of the non-alcoholic steatohepatitis diseases, is a representative example of liver disease accompanied by liver cell necrosis, inflammation, and fibrosis. The composition can suppress liver inflammation and fibrosis and thereby shows an effect on non-alcoholic steatohepatitis (NASH), and specifically, can show an effect on non-alcoholic steatohepatitis (NASH) accompanied by fatty liver, liver fibrosis, or cirrhosis; or liver cancer caused by non-alcoholic steatohepatitis (NASH), but the diseases are not limited thereto.

As used herein, "liver fibrosis" refers to the formation of excessive fibrous connective tissue in organs or tissues during a reparative or responsive process as a result of a wound healing process for repeated liver damage. Chronicity and aggravation of liver inflammation are known to be the cause of the occurrence of liver fibrosis. Liver fibrosis is known to be reversible (unlike cirrhosis), to be composed of thin fibrils, and to have no nodule formation. Once the cause of liver damage ceases, the recovery of normal liver may be possible. However, when the liver fibrosis process is repeated continuously, the crosslinking between the extra cellular matrices (ECMs) increases, thereby resulting in the progression of irreversible cirrhosis with nodules.

The composition according can show a preventive or therapeutic effect on liver fibrosis, and specifically on liver fibrosis accompanied by non-alcoholic steatohepatitis (NASH), but the effects are not limited thereto.

The composition, when administered to a subject, can show a preventive or therapeutic effect on liver fibrosis in the subject administered with the composition by reducing the blood levels of TIMP-1 and/or hyaluronic acid, but the effects are not limited thereto.

Specifically, the peptide or a conjugate thereof can show an effect on liver fibrosis, and specifically, the effect may be to prevent or treat liver fibrosis by reducing the enhanced liver fibrosis (ELF) score.

The ELF score (the enhanced liver fibrosis score) is a score that confirms the degree of healing of liver fibrosis, and it can be calculated by the following equation. The ELF score can be calculated by the following equation after measuring the concentrations of hyaluronic acid (HA), N-terminal propeptide of procollagen type III (PIIINP), and tissue inhibitor of metalloproteinase-1 (TIMP-1) in blood samples:

$$\text{ELF Score} = 2.278 + 0.851\ \ln(\text{CHA}) + 0.751\ \ln(\text{CPIIINP}) + 0.394\ \ln(\text{CTIMP-1})$$

The reduction of the ELF score above may be a reduction of about 10% to about 100%, about 10% to about 95%, about 10% to about 90%, about 10% to about 80%, about 10% to about 70%, about 10% to about 60%, about 10% to about 50%, or about 14% to about 30%, compared to the group not administered with the peptide or a long-acting conjugate thereof, but the reduction of the ELF score is not limited thereto.

The composition may prevent or treat liver fibrosis by reducing the ELF score of the subject administered with the composition to about 9.8 or below, about 9.8 or below, about 9.7 or below, about 9.6 or below, about 9.5 or below, about 9.4 or below, about 9.3 or below, about 9.2 or below, or about 9.1 or below, but the reduction of the ELF score is not limited thereto.

In the present disclosure "cholestasis" refers to a condition in which the bile flow from the liver to the duodenum is slowed or blocked, and "cholestasis liver disease" means that bile formation in the liver is impaired by conditions such as various diseases, expanded jugular nutrition, or side effects of specific drugs (*e.g.*, some antibiotics). Common signs of cholestasis include fatigue, pruritus (itching), jaundice, and xanthoma (deposition of subcutaneous cholesterol-rich substances). The effects of cholestasis are extreme and broad, causing exacerbation of liver disease to a systemic disease, liver decompensation, and the need for liver transplantation. Causes of cholestasis liver disease may include acute hepatitis, inflammation of the bile ducts, *etc.*

The cholestasis liver disease may include primary biliary cholangitis (PBC), primary sclerosing cholangitis (PSC), progressive familial intrahepatic cholestasis (PFIC), and Alagille syndrome (AS), but the cholestasis liver disease is not limited thereto.

Primary biliary cirrhosis, which is also known as primary biliary cholangitis (PBC), is a cryptogenic chronic cholestasis liver disease. Progressive bile duct damage due to portal and periportal inflammation can cause progressive fibrosis and ultimate cirrhosis. Thus far, immunological, genetic, and environmental factors are known as potential causes of primary biliary cirrhosis. Primary biliary cirrhosis mostly occurs in middle-aged women, and symptoms such as fatigue, itching, or unidentified hyperlipidemia may also appear in the early onset of primary biliary cirrhosis.

At present, primary biliary cirrhosis is understood to be as an immune-mediated disease, and specifically, the immunohistochemical staining of T lymphocytes in the portal and periportal regions shows CD4-positive and CD8-negative T cells.

In addition, abnormal suppressor T-cell activity was reported in asymptomatic first-grade relatives of affected subjects. It was reported that interleukins can have a role in the pathogenesis of PBC by contributing to altered immune functions and fibrosis (G.J. Webb et al., J. Autoimmunity, 2015 Nov; 64: 42-52).

The method for treating PBC is a bile acid therapy using ursodeoxycholic acid (UDSA) and obeticholic acid (OCA). The action mechanism of the two drugs in PBC is associated with their ability to activate FXR and TGFR-5 to exert their anti-inflammatory effects. However, a sufficient biochemical response was not achieved in about 40% of the patients treated with UDCA.

Primary sclerosing cholangitis (PSC) is a cryptogenic chronic cholestasis liver disease caused by inflammation and fibrosis of the intrahepatic and extrahepatic bile ducts. Specifically, it is an inflammatory disease of the bile ducts and biliary tract, and once the disease progresses, fibrosis occurs and the bile duct wall becomes thickened, thereby narrowing the bile ducts.

The causes of the disease have not yet been identified, but it appears that a combination of various factors such as genetic factors, environmental factors, and related immune responses may be a possible cause.

When the results of liver function tests through blood show an increase in alkaline phosphatase levels, an increase in aminotransferase levels, and an indication of gamma globulinemia, the subject is diagnosed as having primary sclerosing cholangitis.

The method for treating PSC has not been clearly reported thus far, and liver transplant surgery is the only treatment that can treat PSC fundamentally.

Accordingly, there is still a need to develop a drug capable of treating PBS and PSC without side effects while securing the patient’s convenience.

The "liver cirrhosis" of the present disclosure is a chronic disease that occurs with repeated increasing of the regeneration of liver cells and fibrous tissue, it is pathologically accompanied by necrosis, inflammation, and fibrosis, and it progresses into cirrhosis complications (*e.g.,* liver decompensation) and diseases (*e.g.,* liver cancer), eventually leading to death. In particular, since liver cirrhosis can be discovered only after considerable progress due to the absence of awareness of one’s own symptoms in the early stages of the disease, it is required that liver fibrosis, which is a condition before it evolves into cirrhosis, *etc.,* be treated promptly. The composition may show a preventive or therapeutic effect on liver cirrhosis, and specifically on liver cirrhosis accompanied by non-alcoholic steatohepatitis (NASH), but the effects of the composition are not limited thereto.

In the present disclosure, "liver decompensation" refers to a condition in which liver function is weakened and

the liver cannot perform protein synthesis and metabolic functions as normal physiological functions due to viral hepatitis, cirrhosis, liver damage by drugs or alcohol, or liver disease. Liver decompensation is divided into acute liver decompensation and chronic liver decompensation according to the progression rate, and it is known to cause various complications. Since the composition shows effects such as inhibition of inflammation and fibrosis, it may show a preventive or therapeutic effect on liver decompensation.

In the present disclosure, "liver cancer (hepatocellular carcinoma)" refers to a malignant tumor originating from liver cells, and it can be classified as primary liver cancer (hepatocellular carcinoma), which occurs in the liver cells themselves, and metastatic liver cancer, in which cancers of other tissues have metastasized to the liver, and about 90% or more of liver cancer is primary liver cancer. Major causes are alcohol, smoking, obesity, *etc.,* in addition to hepatitis and chronic liver disease.

The composition may show a preventive or therapeutic effect on liver cancer, and specifically liver cancer caused by non-alcoholic steatohepatitis (NASH), but the effects of the composition are not limited thereto.

The models used in the examples are known as: a non-alcoholic steatohepatitis (NASH) model induced by the MCD diet; and fatty liver and steatohepatitis models induced by the AMLN diet. In addition, the AMLN/TAA mouse model is known to be used as a liver fibrosis or non-alcoholic steatohepatitis (NASH) model. The above model is a model used in various studies associated with liver disease, and in the examples, the effect of the peptide according to the present invention (a triple agonist) or a long-acting conjugate thereof was confirmed in each model, which suggests that the peptide (a triple agonist) or a long-acting conjugate thereof is useful for the prevention or treatment of liver disease (*e.g.*, hepatitis, liver fibrosis, simple steatosis, non-alcoholic fatty liver (NAFL), non-alcoholic steatohepatitis (NASH), *etc.*). In addition, in the examples, the effect of improving the long-acting conjugate of a triple agonist was confirmed in the PBC and/or PSC models, and the effect on cholestasis liver disease was also confirmed.

The composition may be characterized in that there is no weight gain or a relatively low degree of weight gain, which is a side effect of the conventional therapeutic agent for liver disease.

The composition may prevent or treat liver disease by performing one or more of the following characteristics of (a) to (k), but the characteristics to be performed are not limited thereto.

(a) a decrease in NAS values (non-alcoholic steatohepatitis disease (NAFLD) Activity Score);
(b) a decrease in levels of triglycerides in the liver;
(c) a decrease in levels of cholesterol in the blood;
(d) a decrease in the steatosis score;
(e) a decrease in levels of TNF-$\alpha$, MCP-1, and IL-6 in liver tissue;
(f) a decrease in the inflammation score of the liver;
(g) a decrease in the parenchymal necrosis score;
(h) a decrease in the bile duct hyperplasia score;
(i) a decrease in the enhanced liver fibrosis (ELF) score;
(j) a decrease in the blood concentration of TIMP-1 and/or hyaluronic acid (*i.e.,* liver fibrosis markers); and
(k) a decrease in the fibrosis score.

As used herein, the term "prevention" refers to all activities that inhibit or delay the occurrence of liver disease by administering the above peptide or the composition containing the peptide, and the term "treatment" refers to all activities that improve or advantageously change the symptoms of liver disease by administering the above peptide or the composition containing the peptide.

The pharmaceutical composition may further contain a pharmaceutically acceptable excipient, carrier, or diluent. The pharmaceutically acceptable excipient, carrier, or diluent may be one that is not naturally occurring.

As used herein, the term "pharmaceutically acceptable" refers to the properties of having a sufficient amount to show a therapeutic effect and not causing adverse effects, and may be easily determined by those skilled in the art based on the factors well known in the medical field, such as the kind of disease, age, body weight, health status, sex, drug sensitivity of a patient, administration route, administration method, administration frequency, duration of treatment, a drug(s) to be mixed or administered simultaneously, *etc.*

The pharmaceutical composition containing the peptide may further contain a pharmaceutically acceptable excipient. The pharmaceutically acceptable excipient may include, for oral administration, a binder, a lubricant, a disintegrant, a solubilizing agent, a dispersant, a stabilizing agent, a suspending agent, a coloring agent, a fragrance, *etc.*; for injections, a buffering agent, a preservative, an analgesic, a solubilizing agent, an isotonic agent, a stabilizing agent, *etc.,* which may be combined to be used; and for topical administrations, a base, an excipient, a lubricant, a preservative, *etc.*

The formulation type of the composition may be prepared variously by being combined with a pharmaceutically acceptable excipient described above. For example, for oral administration, the composition may be formulated into tablets, troches, capsules, elixirs, suspensions, syrups, wafers, *etc.,* and for injections, the composition may be formulated

into unit-dose ampoules or multi-dose containers. The composition may also be formulated into solutions, suspensions, tablets, pills, capsules, sustained-release formulations, *etc.*

Meanwhile, examples of suitable carriers, excipients, and diluents may include lactose, dextrose, sucrose, sorbitol, mannitol, xylitol, erythritol, maltitol, starch, acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, *etc.* Additionally, the composition may further contain a filler, an anticoagulant, a lubricant, a humectant, a fragrance, a preservative, *etc.*

Additionally, the pharmaceutical composition may have any one formulation type selected from the group consisting of tablets, pills, powders, granules, capsules, suspensions, liquid medicine for internal use, emulsions, syrups, sterile aqueous solutions, non-aqueous solvents, lyophilized formulations, and suppositories.

Additionally, the composition may be formulated into a preparation of a unit dosage form suitable for the administration into a patient's body, and may specifically be formulated into a preparation useful for peptide drugs according to the conventional method in the pharmaceutical field so as to be administered by an oral or parenteral route (including skin, intravenous, intramuscular, intraarterial, intramedullary, intrathecal, intraventricular, pulmonary, transdermal, subcutaneous, intraperitoneal, intranasal, intragastrical, topical, sublingual, vaginal, or rectal route, but the administration routes are not limited thereto.

Additionally, the conjugate may be used by being mixed with various pharmaceutically acceptable carriers such as physiological saline or organic solvents. To increase stability or absorptivity, carbohydrates (*e.g.,* glucose, sucrose, or dextrans), antioxidants (*e.g.,* ascorbic acid or glutathione), chelating agents, low-molecular weight proteins, or other stabilizers, *etc.* may be used as pharmaceutical drugs.

The administration dose and frequency of the pharmaceutical composition are determined by the type of active ingredient(s), together with various factors, such as the disease to be treated, administration route, patient's age, sex, and body weight, severity of the disease, *etc.* Specifically, the composition may be one which contains a peptide comprising an amino acid sequence of any one of SEQ ID NOS: 1 to 102 (not claimed) or a long-acting conjugate containing the peptide in a pharmaceutically effective amount.

Containing the peptide or a long-acting conjugate thereof in a pharmaceutically effective amount refers to a level at which the desired pharmacological activity (*e.g.*, prevention, improvement, or treatment of liver disease) can be obtained by the peptide or a long-acting conjugate thereof, and in addition, may refer to a level at which toxicities or adverse effects do not occur or occur at an insignificant level in the subject to be administered, or may refer to a pharmaceutically acceptable level, but the level is not limited thereto. The pharmaceutically effective amount as such may be determined by comprehensively considering the number of administration, patient, formulations, *etc.*

The total effective amount of the composition may be administered to a patient in a single dose or may be administered for a long period of time in multiple doses according to a fractionated treatment protocol. In the pharmaceutical composition, the content of active ingredient(s) may vary depending on the severity of the disease. Specifically, the total daily dose of the conjugate may be about 0.0001 mg to 500 mg per 1 kg of the body weight of a patient. However, the effective dose of the conjugate is determined considering various factors including patient's age, body weight, health conditions, sex, disease severity, diet, and excretion rate, as well as administration route and treatment frequency of the pharmaceutical composition. In this respect, those skilled in the art may easily determine the effective dose suitable for a particular use of the pharmaceutical composition. The pharmaceutical composition is not particularly limited to the formulation type and administration route and mode, as long as it shows the effects of the present disclosure.

The pharmaceutical composition has excellent *in vivo* duration of efficacy and titer, and thus, the number and frequency of administration of the pharmaceutical preparation can be significantly reduced.

To achieve the objects of the present disclosure, the present disclosure also provides, but does not claim, a method for the prevention or treatment of liver disease, which includes administering the peptide or a composition containing the peptide to a subject in need thereof.

The peptide or a composition containing the same, liver disease, prevention, and treatment are as described above.

In the present disclosure, the subject refers to a subject suspected of having a liver disease, and the subject suspected of having a liver disease refers to mammals including humans, rats, cattle, *etc.,* which have or are at risk of developing the liver disease, but any subject which can be treated with the conjugate or the composition containing the conjugate is included without limitation.

As used herein, the term "administration" refers to the introduction of a particular material into a subject by any appropriate method, and the administration route of the composition may be any conventional route that enables delivery of the composition to the target (e.g., intraperitoneal administration, intravenous administration, intramuscular administration, subcutaneous administration, intradermal administration, oral administration, topical administration, intranasal administration, intrapulmonary administration, intrarectal administration, *etc.*), but the administration route is not limited thereto.

The method may include administering a pharmaceutical composition containing the peptide in a pharmaceutically effective amount.

An appropriate total daily dose of the pharmaceutical composition may be determined within the scope of correct medical judgment by a practitioner, and the pharmaceutical composition may be administered once or several times in divided doses. However, it is preferred that the specific therapeutically effective dose of the pharmaceutical composition for any particular patient be applied differently depending on the kind and degree of responses to be achieved, specific compositions including whether other agents are occasionally used therewith, the patient's age, body weight, health conditions, sex and diet, administration time, administration route, excretion rate of the composition, duration of treatment, other drugs used in combination or simultaneously with the specific compositions, and similar factors well known in the medical field.

To achieve the objects of the present disclosure, the disclosure also provides a use of the peptide or a composition containing the peptide for the prevention or treatment of liver disease.

To achieve the objects of the present disclosure, the disclosure also provides a use of the peptide or a composition containing the peptide in the preparation of a medicament for the prevention or treatment of liver disease.

The peptide or a composition containing the same, liver disease, prevention, and treatment are as described above.

Hereinafter, the present invention will be described in more detail with reference to the following Examples.

## Example 1: Measurement of *in vitro* activities of triple agonists and long-acting conjugates thereof

### Example 1-1: Preparation of triple agonists

Triple agonists showing activities to all of GLP-1, GIP, and glucagon receptors were prepared, and their sequences are shown in Table 1 below.

[Table 1]

| SEQ ID NO | Sequence | Information |
|---|---|---|
| 1 | HXQGTFTSDVSSYLDGQAAKEFIAWLVKGC | - |
| 2 | HXQGTFTSDVSSYLDGQAQKEFIAWLVKGC | - |
| 3 | H X Q G T F T S D V S S Y L L G Q A A K Q F I A W L V K G G G P S S G A P P P S C | - |
| 4 | HXQGTFTSDVSSYLLGQQQKEFIAWLVKGC | - |
| 5 | H X Q G T F T S D V S S Y L L G Q Q Q K E F I A W L V K G G G P S S G A P P P S C | - |
| 6 | HXQGTFTSDVSSYLDGQAAKEFVAWLLKGC | - |
| 7 | HXQGTFTSDVSKYLDGQAAKEFVAWLLKGC | - |
| 8 | HXQGTFTSDVSKYLDGQAAQEFVAWLLKGC | - |
| 9 | HXQGTFT SDV SKYLDGQAAQEF VAWLLAGC | - |
| 10 | H X Q G T F T S D V S K Y L D G Q A A Q E F V A W L L A G G G P S S G A P P P S C | - |
| 11 | CA G E G T F T S D L S K Y L D S R R Q Q L F V Q W L K A G G P S S G A P P P S H G | - |
| 12 | CA G E G T F I S D L S K Y M D E Q A V Q L F V E W L M A G G P S S G A P P P S H G | - |
| 13 | CA G E G T F I S D Y S I Q L D E I A V Q D F V E W L L A Q K P S S G A P P P S H G | - |
| 14 | CA G Q G T F T S D Y S I Q L D E I A V R D F V E W L K N G G P S S G A P P P S H G | - |

(continued)

| SEQ ID NO | Sequence | Information |
|---|---|---|
| 15 | CA G Q G T F T S D L S K Q M D E E A V R L F I E W L K N G G P S S G A P P P S H G | - |
| 16 | CA G Q G T F T S D L S K Q M D S E A Q Q L F I E W L K N G G P S S G A P P P S H G | - |
| 17 | CA G Q G T F T S D L S K Q M D E E R A R E F I E W L L A Q K P S S G A P P P S H G | - |
| 18 | CA G Q G T F T S D L S K Q M D S E R A R E F I E W L K N T G P S S G A P P P S H G | - |
| 19 | CA G Q G T F T S D L S I Q Y D S E H Q R D F I E W L K D T G P S S G A P P P S H G | - |
| 20 | CA G Q G T F T S D L S I Q Y E E E A Q Q D F V E W L K D T G P S S G A P P P S H G | - |
| 21 | Y X Q G T F T S D Y S K Y L D E C R A K E F V Q W L L D H H P S S G Q P P P S | Ring formation |
| 22 | Y X Q G T F T S D Y S K C L D E K R A K E F V Q W L L D H H P S S G Q P P P S | Ring formation |
| 23 | Y X Q G T F T S D Y S K Y L D E C R A K E F V Q W L L A Q K G K K N D W K H N I T | Ring formation |
| 24 | Y X Q G T F T S D Y S K Y L D E C R A K E F V Q W L K N G G P S S G A P P P S | Ring formation |
| 25 | H X Q G T F T S D C S K Y L D E R A A Q D F V Q W L L D G G P | - |

(continued)

| SEQ ID NO | Sequence | Information |
|---|---|---|
| | S S G A P P P S | |
| 26 | H X Q G T F T S D C S K Y L D S R A A Q D F V Q W L L D G G P S S G A P P P S | - |
| 27 | H X Q G T F T S D Y S K Y L D E R A C Q D F V Q W L L D Q G G P S S G A P P P S | - |
| 28 | H X Q G T F T S D Y S K Y L D E K R A Q E F V C W L L A Q K G K K N D W K H N I T | - |
| 29 | HXQGTFTSDYSKYLDEKAAKEFVQWLLNTC | Ring formation |
| 30 | HXQGTFTSDYSKYLDEKAQKEFVQWLLDTC | Ring formation |
| 31 | HXQGTFTSDYSKYLDEKACKEFVQWLLAQ | Ring formation |
| 32 | H X Q G T F T S D Y S K Y L D E K A C K D F V Q W L L D G G P S S G A P P P S | Ring formation |
| 33 | HXQGTFTSDYSIAMDEIHQKDFVNWLLAQKC | Ring formation |
| 34 | HXQGTFTSDYSKYLDEKRQKEFVNWLLAQKC | Ring formation |
| 35 | HXQGTFTSDYSIAMDEIHQKDFVNWLLNTKC | Ring formation |
| 36 | H X Q G T F T S D Y S K Y L C E K R Q K E F V Q W L L N G G P S S G A P P P S G | Ring formation |
| 37 | H X Q G T F T S D Y S K Y L D E C R Q K E F V Q W L L N G G P S S G A P P P S G | Ring formation |
| 38 | CA X Q G T F T S D K S S Y L D E R A A Q D F V Q W L L D G G P S S G A P P P S S | - |

(continued)

| SEQ ID NO | Sequence | Information |
|---|---|---|
| 39 | H X Q G T F T S D Y S K Y L D G Q H A Q C F V A W L L A G G G P S S G A P P P S | - |
| 40 | H X Q G T F T S D K S K Y L D E R A C Q D F V Q W L L D G G P S S G A P P P S | - |
| 41 | H X Q G T F T S D K S K Y L D E C A A Q D F V Q W L L D G G P S S G A P P P S | - |
| 42 | Y X Q G T F T S D Y S K Y L D E K R A K E F V Q W L L D H H P S S G Q P P P S C | Ring formation |
| 43 | Y X Q G T F T S D Y S K Y L D E K R A K E F V Q W L L D H H C S S G Q P P P S | Ring formation |
| 44 | H G Q G T F T S D C S K Q L D G Q A A Q E F V A W L L A G G P S S G A P P P S | - |
| 45 | H G Q G T F T S D C S K Y M D G Q A A Q D F V A W L L A G G P S S G A P P P S | - |
| 46 | H G Q G T F T S D C S K Y L D E Q H A Q E F V A W L L A G G P S S G A P P P S | - |
| 47 | H G Q G T F T S D C S K Y L D G Q R A Q E F V A W L L A G G P S S G A P P P S | - |
| 48 | H G Q G T F T S D C S K Y L D G Q R A Q D F V N W L L A G G P S S G A P P P S | - |
| 49 | CAXQGTFTSDYSICMDEIHQKDFVNWLLNTK | Ring formation |

(continued)

| SEQ ID NO | Sequence | Information |
|---|---|---|
| 50 | HXQGTFTSDYSKYLDEKRAKEFVQWLLDHHP SSGQPPPSC | Ring formation |
| 51 | HXQGTFTSDYSKYLDEKRQKEFVQWLLNTC | Ring formation |
| 52 | HXQGTFTSDYSKYLDEKRQKEFVQWLLDTC | Ring formation |
| 53 | HXEGTFTSDYSIAMDEIHQKDFVNWLLAQC | Ring formation |
| 54 | HXEGTFTSDYSIAMDEIHQKDFVDWLLAEC | Ring formation |
| 55 | HXQGTFTSDYSIAMDEIHQKDFVNWLLAQC | Ring formation |
| 56 | HXQGTFTSDYSKYLDEKRQKEFVNWLLAQC | Ring formation |
| 57 | HXQGTFTSDYSIAMDEIHQKDFVNWLLNTC | Ring formation |
| 58 | HXQGTFTSDYSKYLDEKRQKEFVQWLLNTKC | Ring formation |
| 59 | CAXQGTFTSDYSICMDEKHQKDFVNWLLNTK | Ring formation |
| 60 | CAXQGTFTSDYSIAMDEKHCKDFVNWLLNTK | Ring formation |
| 61 | CAXQGTFTSDYSIAMDEIACKDFVNWLLNTK | Ring formation |
| 62 | CAXQGTFTSDKSKYLDERAAQDFVQWLLDGG PSSGAPPPS | - |
| 63 | CAXQGTFTSDCSKYLDERAAQDFVQWLLDGGP SSGAPPPS | - |
| 64 | YXQGTFTSDYSKYLDECAAKEFVQWLLDHHP SSGQPPPS | Ring formation |
| 65 | HXQGTFTSDYSKCLDEKRAKEFVQWLLDHHPS SGQPPPS | Ring formation |

(continued)

| SEQ ID NO | Sequence | Information |
|---|---|---|
| 66 | Y X Q G T F T S D Y S K Y L D E C R A K D F V Q W L L D H H P S S G Q P P P S | Ring formation |
| 67 | Y X Q G T F T S D Y S K Y L D E C A A K D F V Q W L L D H H P S S G Q P P P S | Ring formation |
| 68 | Y X Q G T F T S D Y S K C L D E K A A K E F V Q W L L D H H P S S G Q P P P S | Ring formation |
| 69 | Y X Q G T F T S D Y S K C L D E R A A K E F V Q W L L D H H P S S G Q P P P S | Ring formation |
| 70 | Y X Q G T F T S D Y S K C L D E K R A K D F V Q W L L D H H P S S G Q P P P S | Ring formation |
| 71 | Y X Q G T F T S D Y S K Y L D E R A C K D F V Q W L L D H H P S S G Q P P P S | Ring formation |
| 72 | Y X Q G T F T S D C S K Y L D E R A A K D F V Q W L L D H H P S S G Q P P P S | Ring formation |
| 73 | CA X Q G T F T S D Y S K Y L D E C R A K E F V Q W L L D H H P S S G Q P P P S | Ring formation |
| 74 | CA X Q G T F T S D Y S K C L D E K R A K E F V Q W L L D H H P S S G Q P P P S | Ring formation |
| 75 | Y X Q G T F T S D Y S K Y L D E K A A K E F V Q W L L D H H P S S G Q P P P S C | Ring formation |

(continued)

| SEQ ID NO | Sequence | Information |
|---|---|---|
| 76 | YXQGTFTSDYSKYLDEKRAKDFVQWLLDHHPSSGQPPPSC | Ring formation |
| 77 | YXQGTFTSDYSKYLDEKAAKDFVQWLLDHHPSSGQPPPSC | Ring formation |
| 78 | HXQGTFTSDYSKYLDEKRQKEFVQWLLDTKC | Ring formation |
| 79 | HXEGTFTSDYSIAMDEIHQKDFVNWLLAQKC | Ring formation |
| 80 | HXEGTFTSDYSIAMDEIHQKDFVDWLLAEKC | Ring formation |
| 81 | CAXQGTFTSDYSKYLDEKRQKEFVQWLLNTC | Ring formation |
| 82 | CAXQGTFTSDYSKYLDEKRQKEFVQWLLDTC | Ring formation |
| 83 | CAXEGTFTSDYSIAMDEIHQKDFVNWLLAQC | Ring formation |
| 84 | CAXEGTFTSDYSIAMDEIHQKDFVDWLLAEC | Ring formation |
| 85 | CAXQGTFTSDYSIAMDEIHQKDFVNWLLAQC | Ring formation |
| 86 | CAXQGTFTSDYSKYLDEKRQKEFVNWLLAQC | Ring formation |
| 87 | CAXQGTFTSDYSIAMDEIHQKDFVNWLLNTC | Ring formation |
| 88 | CAXQGTFTSDYSKYLDEKRQKEFVQWLLNTKC | Ring formation |
| 89 | CAXQGTFTSDYSKYLDEKRQKEFVQWLLDTKC | Ring formation |
| 90 | CAXEGTFTSDYSIAMDEIHQKDFVNWLLAQKC | Ring formation |
| 91 | CAXEGTFTSDYSIAMDEIHQKDFVDWLLAEKC | Ring formation |
| 92 | CAXQGTFTSDYSIAMDEIHQKDFVNWLLAQKC | Ring formation |
| 93 | CAXQGTFTSDYSKYLDEKRQKEFVNWLLAQK C | Ring formation |
| 94 | CAXQGTFTSDYSIAMDEIHQKDFVNWLLNTKC | Ring formation |
| 95 | YXQGTFTSDYSKYLDEKRAKEFVQWLLCHHPSSGQPPPS | Ring formation |

(continued)

| SEQ ID NO | Sequence | Information |
|---|---|---|
| 96 | Y X Q G T F T S D Y S K Y L D E K R A K E F V Q W L L D H C P S S G Q P P P S | Ring formation |
| 97 | Y X Q G T F T S D Y S K Y L D E K R A K E F V Q W L L D C H P S S G Q P P P S | Ring formation |
| 98 | Y X Q G T F T S D Y S K A L D E K A A K E F V N W L L D H H P S S G Q P P P S C | Ring formation |
| 99 | Y X Q G T F T S D Y S K A L D E K A A K D F V N W L L D H H P S S G Q P P P S C | Ring formation |
| 100 | Y X Q G T F T S D Y S K A L D E K A A K E F V Q W L L D Q H P S S G Q P P P S C | Ring formation |
| 101 | Y X Q G T F T S D Y S K A L D E K A A K E F V N W L L D Q H P S S G Q P P P S C | Ring formation |
| 102 | Y X Q G T F T S D Y S K A L D E K A A K D F V N W L L D Q H P S S G Q P P P S C | Ring formation |

In the sequences described in Table 1, the amino acids indicated by X represent aminoisobutyric acid (Aib), which is a non-natural amino acid, and the underlined amino acids represent the formation of a ring between the underlined amino acids. Additionally, in Table 1, CA represents 4-imidazoacetyl and Y represents tyrosine.

Example 1-2: Preparation of long-acting conjugate of triple agonists

For the pegylation of the cysteine residue of triple agonists (SEQ ID NOS: 21, 22, 42, 43, 50, 77, and 96) of Example 1 using PEG (10 kDa) having a maleimide group and an aldehyde group at each ends, i.e., maleimide-PEG-aldehyde (10 kDa, NOF, Japan), the triple agonists and the maleimide-PEG-aldehyde were reacted at a molar ratio of 1:1 to 3, at a protein concentration of 1 mg/mL to 5 mg/mL at low temperature for 0.5 to 3 hours. In particular, the reaction was performed in an environment in which 20% to 60% isopropanol was added to 50 mM Tris buffer (pH 7.5). Upon completion of the reaction, the reactants were applied to SP sepharose HP (GE Healthcare, USA) to purify the triple agonists which were mono-pegylated on cysteine.
Then, the purified mono-pegylated triple agonists and an immunoglobulin Fc were reacted at a molar ratio of 1:1 to 5, at a protein concentration of 10 mg/mL to 50 mg/mL at 4°C to 8°C for 12 to 18 hours. The reaction was performed in an environment in which 10 mM to 50 mM sodium cyanoborohydride (NaCNBH3; a reducing agent) and 10% to 30% isopropanol were added to 100 mM potassium phosphate butter (pH 6.0). Upon completion of the reaction, the reactants were applied to the Butyl sepharose FF purification column (GE Healthcare, USA) and Source ISO purification column (GE Healthcare, USA) to purify the conjugates including the triple agonists and the immunoglobulin Fc.
After the preparation, the purity analyzed by reverse phase chromatography, size exclusion chromatography, and ion exchange chromatography was shown to be 95% or higher.
In particular, the conjugate in which the triple agonist of SEQ ID NO: 21 and an immunoglobulin Fc were linked through PEG was named as a "conjugate including the triple agonist of SEQ ID NO: 21 and an immunoglobulin Fc" or "long-acting conjugate of SEQ ID NO: 21", and these can be used interchangeably.
In particular, the conjugate in which the triple agonist of SEQ ID NO: 22 and an immunoglobulin Fc were linked by PEG was named as "the conjugate including the triple agonist of SEQ ID NO: 22 and an immunoglobulin Fc" or "long-acting conjugate of SEQ ID NO: 22", and these can be used interchangeably.
In particular, the conjugate in which the triple agonist of SEQ ID NO: 42 and an immunoglobulin Fc were linked by PEG was named as "the conjugate including the triple agonist of SEQ ID NO: 42 and an immunoglobulin Fc" or "long-acting conjugate of SEQ ID NO: 42", and these can be used interchangeably.
In particular, the conjugate in which the triple agonist of SEQ ID NO: 43 and an immunoglobulin Fc were linked by PEG was named as "the conjugate including the triple agonist of SEQ ID NO: 43 and an immunoglobulin Fc" or "long-acting conjugate of SEQ ID NO: 43", and these can be used interchangeably.
In particular, the conjugate in which the triple agonist of SEQ ID NO: 50 and an immunoglobulin Fc were linked by PEG was named as "the conjugate including the triple agonist of SEQ ID NO: 50 and an immunoglobulin Fc" or "long-acting conjugate of SEQ ID NO: 50", and these can be used interchangeably.
In particular, the conjugate in which the triple agonist of SEQ ID NO: 77 and an immunoglobulin Fc were linked by PEG was named as "the conjugate including the triple agonist of SEQ ID NO: 77 and an immunoglobulin Fc" or "long-acting conjugate of SEQ ID NO: 77", and these can be used interchangeably.
In particular, the conjugate in which the triple agonist of SEQ ID NO: 96 and an immunoglobulin Fc were linked by PEG was named as "the conjugate including the triple agonist of SEQ ID NO: 96 and an immunoglobulin Fc" or "long-acting conjugate of SEQ ID NO: 96", and these can be used interchangeably.

Example 1-3: Measurement of *in vitro* activities of triple agonists and long-acting conjugates thereof

The activities of the triple agonists and long-acting conjugates thereof prepared in Examples 1-1 and 1-2 were measured by a method of measuring *in vitro* cellular activities using cell lines where a GLP-1 receptor, a glucagon (GCG) receptor, and a GIP receptor are transformed, respectively.
Each of the cell lines above is one in which the genes for a human GLP-1 receptor, a human GCG receptor, and a human GIP receptor are transformed into Chinese hamster ovary (CHO), respectively, to be expressed therein, and is thus suitable for the measurement of the activities of GLP-1, GCG, and GIP. Accordingly, the activity for each part was measured using the respective transformed cell line.
For the measurement of the GLP-1 activities of the triple agonists and long-acting conjugates thereof prepared in Examples 1-1 and 1-2, human GLP-1 was subjected to a 4-fold serial dilution from 50 nM to 0.000048 nM, and the triple agonists and long-acting conjugates thereof prepared in Examples 1-1 and 1-2 were subjected to a 4-fold serial dilution from 400 nM to 0.00038 nM. The culture solution was removed from the cultured CHO cells, in which the human GLP-1 receptor was expressed, and each of the serially diluted materials was added to the CHO cells in an amount of 5 μL, respectively, and a buffer solution containing a cAMP antibody was added thereto in an amount of 5 μL and

cultured at room temperature for 15 minutes. Then, a detection mix containing a cell lysis buffer was added thereto in an amount of 10 μL for the lysis of the cells and reacted at room temperature for 90 minutes. The cell lysates, after completion of the reaction, were applied to the LANCE cAMP kit (PerkinElmer, USA) to calculate the $EC_{50}$ value through accumulated cAMP, and the values were compared with one another. The relative titers compared to human GLP-1 are shown in Tables 2 and 3 below.

For the measurement of the GCG activities of the triple agonists and long-acting conjugates thereof prepared in Examples 1-1 and 1-2, human GCG was subjected to a 4-fold serial dilution from 50 nM to 0.000048 nM, and the triple agonists and long-acting conjugates thereof prepared in Examples 1-1 and 1-2 were subjected to a 4-fold serial dilution from 400 nM to 0.00038 nM. The culture solution was removed from the cultured CHO cells, in which the human GCG receptor was expressed, and each of the serially diluted materials was added to the CHO cells in an amount of 5 μL, respectively, and a buffer solution containing a cAMP antibody was added thereto in an amount of 5 μL and cultured at room temperature for 15 minutes. Then, a detection mix containing a cell lysis buffer was added thereto in an amount of 10 μL for the lysis of the cells and reacted at room temperature for 90 minutes. The cell lysates, after completion of the reaction, were applied to the LANCE cAMP kit (PerkinElmer, USA) to calculate the $EC_{50}$ value through accumulated cAMP, and the values were compared with one another. The relative titers compared to human GCG are shown in Tables 2 and 3 below.

For the measurement of the GIP activities of the triple agonists and long-acting conjugates thereof prepared in Examples 1-1 and 1-2, human GIP was subjected to a 4-fold serial dilution from 50 nM to 0.000048 nM, and the triple agonists and long-acting conjugates thereof prepared in Examples 1-1 and 1-2 were subjected to a 4-fold serial dilution from 400 nM to 0.00038 nM. The culture solution was removed from the cultured CHO cells, in which the human GIP receptor was expressed, and each of the serially diluted materials was added to the CHO cells in an amount of 5 μL, respectively, and a buffer solution containing a cAMP antibody was added thereto in an amount of 5 μL and cultured at room temperature for 15 minutes. Then, a detection mix containing a cell lysis buffer was added thereto in an amount of 10 μL for the lysis of the cells and reacted at room temperature for 90 minutes. The cell lysates, after completion of the reaction, were applied to the LANCE cAMP kit (PerkinElmer, USA) to calculate the $EC_{50}$ value through accumulated cAMP, and the values were compared with one another. The relative titers compared to human GIP are shown in Tables 2 and 3 below.

[Table 2] Relative titer ratio of triple agonists

| | *In vitro* Activity Compared to Native Peptide (%) | | |
|---|---|---|---|
| SEQ ID NO | *vs.* GLP-1 | *vs.* Glucagon | *vs.* GIP |
| 1 | 3.2 | <0.1 | <0.1 |
| 2 | 5.9 | <0.1 | <0.1 |
| 3 | 1.8 | <0.1 | <0.1 |
| 4 | 8.5 | <0.1 | <0.1 |
| 5 | 42.1 | <0.1 | <0.1 |
| 6 | 17.0 | <0.1 | <0.1 |
| 7 | 13.7 | <0.1 | <0.1 |
| 8 | 14.2 | 0.10 | <0.1 |
| 9 | 32.1 | 0.13 | <0.1 |
| 10 | 46.0 | <0.1 | <0.1 |
| 11 | 1.4 | <0.1 | <0.1 |
| 12 | 0.4 | <0.1 | <0.1 |
| 13 | <0.1 | < 0.1 | < 0.1 |
| 14 | 28.0 | < 0.1 | < 0.1 |
| 15 | 79.2 | <0.1 | <0.1 |
| 16 | 2.1 | < 0.1 | < 0.1 |
| 17 | 0.2 | <0.1 | < 0.1 |
| 18 | <0.1 | <0.1 | <0.1 |

(continued)

| | *In vitro* Activity Compared to Native Peptide (%) | | |
|---|---|---|---|
| SEQ ID NO | *vs.* GLP-1 | *vs.* Glucagon | *vs.* GIP |
| 19 | <0.1 | <0.1 | <0.1 |
| 20 | <0.1 | <0.1 | <0.1 |
| 21 | 17.8 | 267 | 22.7 |
| 22 | 20.1 | 140 | 59.7 |
| 23 | 4.01 | 9.3 | <0.1 |
| 24 | 41.2 | 9.3 | < 0.1 |
| 25 | 82.6 | 0.1 | <0.1 |
| 26 | 64.5 | 0.2 | <0.1 |
| 27 | 83.1 | 0.8 | 0.9 |
| 28 | 17.2 | 1.6 | <0.1 |
| 29 | 38.5 | 6.0 | <0.1 |
| 30 | 142 | 0.7 | 0.8 |
| 31 | 135 | 2.2 | 2.4 |
| 32 | 151 | 1.7 | 8.8 |
| 33 | 24.5 | <0.1 | 10.4 |
| 34 | 19.1 | 0.92 | 0.6 |
| 35 | 7.5 | <0.1 | 1.3 |
| 36 | 37.4 | 0.39 | 0.2 |
| 37 | 236 | 6.21 | 2.2 |
| 38 | 2.3 | - | - |
| 39 | 13.9 | 0.53 | <0.1 |
| 40 | 75.2 | <0.1 | <0.1 |
| 41 | 34.3 | <0.1 | <0.1 |
| 42 | 33.9 | 205.8 | 7.8 |
| 43 | 12.6 | 88.4 | 3.70 |
| 44 | 1.3 | <0.1 | <0.1 |
| 45 | 6.6 | < 0.1 | < 0.1 |
| 46 | 1.4 | <0.1 | < 0.1 |
| 47 | 2.4 | <0.1 | < 0.1 |
| 48 | 1.5 | <0.1 | < 0.1 |
| 49 | 29.8 | <0.1 | 3.3 |
| 50 | 67.4 | 50.5 | 2.7 |
| 51 | 14.4 | 2.0 | 0.1 |
| 52 | 44.1 | 7.5 | 0.3 |
| 53 | 161 | 8.4 | 1.3 |
| 54 | 30.6 | 1.4 | 0.1 |
| 55 | 27.1 | 0.7 | 2.4 |

(continued)

| | *In vitro* Activity Compared to Native Peptide (%) | | |
|---|---|---|---|
| SEQ ID NO | *vs.* GLP-1 | *vs.* Glucagon | *vs.* GIP |
| 56 | 57.9 | 4.9 | 0.8 |
| 57 | 11.7 | <0.1 | 0.3 |
| 58 | 39.1 | 2.6 | 0.2 |
| 59 | 40.3 | <0.1 | 4.0 |
| 60 | 106.2 | <0.1 | 8.2 |
| 61 | 59.8 | <0.1 | 2.8 |
| 62 | 5.2 | <0.1 | <0.1 |
| 63 | 15.3 | <0.1 | <0.1 |
| 64 | 64.6 | 60.1 | 92.9 |
| 65 | 95.4 | 25.2 | 11.6 |
| 66 | 15.8 | 172 | 17.2 |
| 67 | 28.5 | 46.2 | 39.8 |
| 68 | 27.9 | 8.8 | 107 |
| 69 | 24.3 | 9.6 | 62.8 |
| 70 | 15.1 | 71.3 | 64.4 |
| 71 | 90.1 | 12.7 | 94.7 |
| 72 | 11.5 | 1.0 | 1.6 |
| 73 | 22.6 | 5.4 | 3.0 |
| 74 | 12.9 | 0.9 | 1.0 |
| 75 | 35.1 | 8.5 | 18.0 |
| 76 | 10.3 | 47.6 | 11.7 |
| 77 | 38.7 | 12.2 | 35.5 |
| 78 | 51.0 | 14.0 | 0.12 |
| 79 | 41.5 | 4.9 | 1.4 |
| 80 | 8.1 | 0.0 | 0.1 |
| 81 | 7.8 | 0.3 | <0.1 |
| 82 | 9.5 | 1.1 | <0.1 |
| 83 | 47.3 | 1.3 | 0.4 |
| 84 | 4.2 | <0.1 | <0.1 |
| 85 | 4.3 | <0.1 | 0.3 |
| 86 | 28.4 | 0.4 | 0.2 |
| 87 | 0.9 | <0.1 | <0.1 |
| 88 | 9.6 | 0.3 | <0.1 |
| 89 | 7.1 | 0.7 | <0.1 |
| 90 | 7.4 | <0.1 | <0.1 |
| 91 | 31.9 | 16.8 | 0.3 |
| 92 | 0.8 | <0.1 | 0.4 |

(continued)

| | *In vitro* Activity Compared to Native Peptide (%) | | |
|---|---|---|---|
| SEQ ID NO | *vs.* GLP-1 | *vs.* Glucagon | *vs.* GIP |
| 93 | 5.7 | 0.3 | 0.7 |
| 94 | 0.5 | <0.1 | <0.1 |
| 95 | 2.1 | 0.4 | <0.1 |
| 96 | 34.4 | 194.8 | 5.2 |
| 97 | 10.5 | 62.8 | 2.6 |
| 98 | 28.1 | 8.2 | 47.1 |
| 99 | 20.9 | 14.9 | 57.7 |
| 100 | 42.2 | 12.7 | 118.5 |
| 101 | 23.2 | 13.9 | 40.1 |
| 102 | 23.3 | 29.5 | 58.0 |

[Table 3] Relative titer ratio of long-acting conjugates of triple agonists

| Long-Acting Conjugate | *In vitro* Activity Compared to Native Peptide (%) | | |
|---|---|---|---|
| | *vs.* GLP-1 | *vs.* Glucagon | *vs.* GIP |
| 21 | 0.1 | 1.6 | 0.2 |
| 22 | 0.1 | 0.9 | 0.5 |
| 42 | 3.1 | 23.1 | 1.2 |
| 43 | 2.1 | 13.5 | 0.6 |
| 50 | 15.4 | 6.9 | 0.7 |
| 77 | 6.7 | 1.7 | 6.6 |
| 96 | 0.3 | 4.0 | 0.3 |

The novel long-acting conjugates of the triple agonists prepared above have the function of triple agonists which can activate all of GLP-1 receptors, GIP receptors, and glucagon receptors, and thus the long-acting conjugates of the triple agonists can be used as a therapeutic material for treating a target disease.

**Example 2: Confirmation of therapeutic effect of triple agonists on metabolic liver disease**

The present inventors attempted to confirm the therapeutic effect of triple agonists on metabolic liver disease.

Example 2-1: Effect of treating NASH in NASH mice induced by MCD dietary intake

First, the effect of long-acting conjugates of triple agonists on non-alcoholic steatohepatitis (NASH) was confirmed as follows.

A mouse model of non-alcoholic steatohepatitis (NASH) was induced by performing dietary intake of a methionine- choline-deficient (MCD) diet for 2 weeks in C57BL/6 mice.

In order to confirm the therapeutic effect of the developed material on the treatment of NASH, the mice were divided into normal mice; NASH-induced mice (an excipient control group), and a group administered with the long-acting conjugate of SEQ ID NO: 42 (0.36 nmol/kg, 0.72 nmol/kg, and 1.44 nmol/kg, Q2D), and the material was subcutaneously administered repeatedly for 4 weeks. After repeated administration for 4 weeks, liver tissue was collected from each mouse by autopsy and subjected to haematoxylin and eosin (H&E) staining so as to evaluate the degree of NASH progression by non-alcoholic steatohepatitis disease activity score (NAS).

As shown in FIG. 1, as a result of the repeated administration of the long-acting conjugate of SEQ ID NO: 42

for 4 weeks, it was confirmed that the NAS value in the liver tissue was significantly reduced (NASH excipient control group = 3.71, group administered with long-acting conjugate of SEQ ID NO: 42 (0.36 nmol/kg) = 2.57, group administered with long-acting conjugate of SEQ ID NO: 42 (0.72 nmol/kg) = 0.43, group administered with long-acting conjugate of SEQ ID NO: 42 (1.44 nmol/kg) = 0).

From the above results, the therapeutic effect on NASH for the long-acting conjugate of SEQ ID NO: 42, which is the conjugate of the representative triple agonist according to the present invention, was confirmed.

Example 2-2: Effect of improving fatty liver in mice induced by AMLN diet

In addition, the present inventors used an AMLN mouse model so as to confirm the effect of the triple agonists on the improvement of fatty liver.

It is known that the AMLN diet has high contents of fat, fructose, and cholesterol, and thus it is known to induce obesity and steatohepatitis when it is fed for a long period of time. Therefore, the AMLN mouse model is used as a model of steatohepatitis.

Mice induced with a 37-week AMLN diet were divided into an excipient control group; a group administered with obeticholic acid (30 mg/kg, QD, oral administration); and a group administered with the long-acting conjugate of SEQ ID NO: 42 (2.6 nmol/kg, Q2D, subcutaneous administration); and they were subjected to repeated administration for 12 weeks. After 12 weeks of repeated administration, liver tissue was collected from each mouse by autopsy, and the efficacy of improving fatty liver was evaluated by measuring the fat content in the liver tissue and through H&E staining.

As a result, it was confirmed that when the long-acting conjugate of SEQ ID NO: 42 was repeatedly administered for 12 weeks, the levels of triglycerides and cholesterol were significantly reduced in the group administered with the long-acting conjugate of SEQ ID NO: 42, compared to those of the excipient control group and the group administered with obeticholic acid. From this result, it was confirmed that the fat content in the liver was reduced by the long-acting conjugate of SEQ ID NO: 42 according to the present invention (FIG. 2).

In addition, in order to further confirm the effect of improving fatty liver according to the administration of the long-acting conjugate of SEQ ID NO: 42, the present inventors examined the changes in steatosis score by administering the long-acting conjugate of SEQ ID NO: 42 in the same manner as described above.

As a result, it was confirmed that when the long-acting conjugate of SEQ ID NO: 42 was administered, the steatosis score (*i.e.,* a value indicating the level of steatosis level) was significantly reduced compared to those of the excipient control group and the group administered with obeticholic acid (FIG. 3).

**Example 3: Confirmation of therapeutic effect of triple agonists on liver fibrosis**

Example 3-1: Confirmation of effect of improving liver fibrosis index in mice with liver fibrosis induced by TAA administration

In order to confirm the effect of improving liver fibrosis by the long-acting conjugates of the triple agonists prepared in Example 1, an AMLN/TAA (thioacetamide) mouse model known as a liver fibrosis model was used. Briefly, C57BL/6 mice were subjected to AMLN dietary intake and TAA administration (50 mg/kg to 400 mg/kg, TIW: 3 times per week) for 16 weeks to induce a model. The animals induced were divided into an excipient control group and a group administered with the long-acting conjugate of SEQ ID NO: 42 (1.3 nmol/kg, Q2D), which is selected as a epresentative triple agonist, and the corresponding material was repeatedly administered subcutaneously during the final 8 weeks of the induction period. The mice fed with only an AMLN diet were used as a negative control. After 8 weeks of repeated administration, the blood concentrations of hyaluronic acid, tissue inhibitor of metalloproteinase-1 (TIMP-1), and N-terminal propeptide of procollagen type III (PIIINP) in the blood samples obtained by blood collection were analyzed, and thereby the enhanced liver fibrosis (ELF) score, which is known as a non-invasive liver fibrosis index, was calculated.

As shown in FIG. 4, it was confirmed that as a result of the repeated administration of the long-acting conjugate of SEQ ID NO: 42 for 8 weeks, the ELF score, which was increased in the AMLN/TAA excipient control group, was significantly reduced.

These results suggest that the triple agonists of the present invention or a long-acting conjugate thereof can significantly lower the ELF score, and thus have a preventive or therapeutic effect on liver fibrosis.

Example 3-2: Confirmation of therapeutic effect on liver fibrosis in mice with liver fibrosis induced by TAA administration

Based on the effect of improving the non-invasive liver fibrosis index confirmed in Example 3-1, an invasive method was applied so as to explicitly evaluate the therapeutic effect of the long-acting conjugates of the triple agonists on liver fibrosis. Briefly, the liver tissue of the mice used in Example 3-1 (8-week repeated administration) was collected by autopsy and then subjected to sirius red staining.

As a result, it was confirmed that the positive area in liver tissue by the sirius red staining was also significantly

reduced by the administration of the long-acting conjugate of the triple agonist (FIG. 5).

These results suggest that the triple agonist of the present invention or a long-acting conjugate thereof has a preventive or therapeutic effect on liver fibrosis.

Example 3-3: Confirmation of effect of improving liver fibrosis in mice with liver fibrosis induced by BDL

In order to confirm the effect of improving liver fibrosis by the long-acting conjugate of SEQ ID NO: 42 confirmed in Examples 3-1 and 3-2, a bile duct ligation (BDL) mouse model, which is known as a liver fibrosis model, was used. Briefly, C57BL/6 mice were anesthetized, and the mice were induced to have cholestasis by suturing the bile duct by surgical therapy, and thereby the mice were induced to have liver fibrosis. The animals induced were divided into an excipient control group and a group administered with a long-acting conjugate of SEQ ID NO: 42 selected as a representative triple agonist (1.3 nmol/kg, Q2D, subcutaneous administration). As a control, mice administered with obeticholic acid (30 mg/kg, QD, oral administration), which is an active pharmaceutical ingredient of Ocaliva®, were used. Drug administration was repeated for 2 weeks starting from the 2nd day after surgery. As a negative control, sham mice were used. Blood concentrations of TIMP-1 and hyaluronic acid, which are index markers for liver fibrosis, were measured using the blood samples obtained by blood collection from mice, which were repeatedly administered with excipient, long-acting conjugate of SEQ ID NO: 42, or obeticholic acid for 2 weeks.

As a result, it was confirmed that the concentrations of TIMP-1 and hyaluronic acid were consistently reduced by the administration of the long-acting conjugate of the triple agonist (FIG. 6).

These results again suggest that the triple agonist of the present invention or a long-acting conjugate thereof has a therapeutic effect on liver fibrosis.

Example 3-4: Confirmation of therapeutic effect on liver fibrosis in mice with liver fibrosis induced by BDL

Based on the effect of improving the non-invasive liver fibrosis index confirmed in Example 3-3, an invasive method was applied so as to explicitly evaluate the therapeutic effect of the long-acting conjugates of the triple agonist on liver fibrosis. Briefly, the liver tissue of the mice used in Example 3-3 (2-week repeated administration) was collected by autopsy and then subjected to sirius red staining. The fibrosis score was measured based on the sirius red staining.

As a result, it was confirmed that when the long-acting conjugate of SEQ ID NO: 42 was repeatedly administered for 2 weeks, the fibrosis score, which was increased in the BDL excipient control group, was significantly decreased (FIGS. 7a and 7b).

The above results suggest that the triple agonist of the present invention or a long-acting conjugate thereof can be used as an agent for the prevention or treatment of liver fibrosis.

**Example 4: Confirmation of effect of triple agonist on liver inflammation**

In order to confirm the therapeutic effect on cholestasis liver disease, which is a liver disease, experiments were performed as follows.

Example 4-1: Confirmation of effect of improving primary biliary cirrhosis (PBC) in mice with PBC induced by BDL

In order to confirm the effect of improving primary biliary cirrhosis (PBC) by the long-acting conjugates of the triple agonists prepared in Example 1, a bile duct ligation (BDL) mouse model, which is known as a PBC model, was used. Briefly, C57BL/6 mice were anesthetized, and the mice were induced to have cholestasis by suturing the bile duct by surgical therapy, and thereby the mice were induced to have liver inflammation.

The animals induced were divided into an excipient control group and a group administered with a long-acting conjugate of SEQ ID NO: 42 (1.3 nmol/kg, Q2D, subcutaneous administration). As a control, mice administered with obeticholic acid (30 mg/kg, QD, oral administration), which is an active pharmaceutical ingredient of Ocaliva® that is commercially available as a therapeutic agent for PBC treatment, were used. Drug administration was repeated for 2 weeks starting from the 2nd day after surgery. As a negative control, sham mice were used. After 2 weeks of repeated administration, the liver tissue was collected from each mouse by autopsy, and the effect of improving liver inflammation was evaluated by H&E staining.

As a result, it was confirmed that when the long-acting conjugate of the triple agonist was repeatedly administered for 2 weeks, the inflammation score, which was increased in the BDL excipient control group, was significantly decreased (FIG. 8).

From the above results, it was confirmed that the long-acting conjugate of the triple agonist has an excellent effect of improving liver inflammation in PBC mice.

Example 4-2: Confirmation of effect of improving primary sclerosing cholangitis (PSC) in mice with PSC induced by BDL

In order to confirm the effect of improving primary sclerosing cholangitis (PSC) by the long-acting conjugates of the triple agonists prepared in Example 1, a bile duct ligation (BDL) mouse model, which is known as a PSC model, was used.

Specifically, C57BL/6 mice were anesthetized, and the mice were induced to have cholestasis by suturing the bile duct by surgical therapy, and thereby the mice were induced to have injuries on the liver and the bile ducts. The animals induced were divided into an excipient control group and a group administered with a long-acting conjugate of SEQ ID NO: 42 (1.3 nmol/kg, Q2D). Repeated subcutaneous administration of the conjugate was performed for 2 weeks starting from the 2$^{nd}$ day after surgery. After 2 weeks of repeated administration, the liver tissue was collected from each mouse by autopsy, and the effect of improving the injuries on the liver and the bile ducts was evaluated by H&E staining.

As a result, it was confirmed that when the long-acting conjugate of the triple agonist was repeatedly administered for 2 weeks, the parenchymal necrosis score by bile reflux, which was increased in the BDL excipient control group, was significantly decreased (FIG. 9).

In addition, as shown in FIG. 10, it was confirmed that the increase in the bile duct hyperplasia score due to the injury on the bile ducts was significantly decreased by the administration of the long-acting conjugate of the triple agonist.

From the above results, it was confirmed that the long-acting conjugate of the triple agonist can improve the injuries on the liver and the bile ducts and alleviate liver inflammation in PSC mice.

Example 4-3: Confirmation of effect of improving liver inflammation in mice administered with TAA

In order to confirm the effect of improving inflammation in the liver by the long-acting conjugates of the triple agonists prepared in Example 1, the present inventors used an AMLN/TAA (thioacetamide) mouse model.

Specifically, C57BL/6 mice were subjected to AMLN dietary intake and TAA administration (50 mg/kg to 400 mg/kg, TIW: 3 times per week) for 16 weeks to induce a model. The animals induced were divided into an excipient control group and a group administered with the long-acting conjugate of SEQ ID NO: 42 (1.3 nmol/kg, Q2D), and the corresponding material was repeatedly administered subcutaneously during the final 8 weeks of the induction period. The mice fed with only AMLN diet were used as a negative control. In addition, the expression level of cytokines in the liver tissue of each mouse collected by autopsy was measured.

Specifically, referring to FIG. 11, it was confirmed that when the expression level of MCP-1 for AMLN (an excipient control group) was set at 1.0, the relative expression level of MCP-1 was 1.506 for AMLN/TAA (an excipient control group) and 0.984 for AMLN/TAA (the long-acting conjugate of the SEQ ID NO: 42); and when the expression level of IL-6 for AMLN (an excipient control group) was set at 1.0, the relative expression level of IL-6 was 1.61 for AMLN/TAA (an excipient control group) and 1.048 for AMLN/TAA (the long-acting conjugate of the SEQ ID NO: 42). It was confirmed that the expression levels of MCP-1 and IL-6 in groups administered with the long-acting conjugate of the triple agonist were reduced by 34.7% and 34.9% relative to that for AMLN/TAA, respectively.

That is, as shown in FIG. 11, it was confirmed that the expression levels of MCP-1 and/or IL-6 in the liver tissue were consistently decreased by the administration of the long-acting conjugate of the triple agonist.

In addition, to further confirm the effect of improving liver inflammation confirmed in FIG. 11, the changes in the level of human necrosis factor-$\alpha$ (TNF-$\alpha$) were measured in a human macrophage cell line (THP-1 cell line).

Specifically, the human macrophage cell line was treated with phorbol 12-myristate 13-acetate (PMA) and allowed to differentiate for 72 hours. Then, the resulting human macrophage cell line was treated with each of the triple agonists of SEQ ID NO: 42, SEQ ID NO: 66, SEQ ID NO: 67, SEQ ID NO: 97, and SEQ ID NO: 100 in a medium to a concentration of 1 $\mu$M, and each was used as a test group. Each of the triple agonists was treated for 48 hours and then treated with lipopolysaccharide (LPS) for 6 hours so as to activate the inflammatory response.

After 12 hours, the changes in the amount of human necrosis factor-$\alpha$ (TNF-$\alpha$), which was secreted in each of the media of test groups treated with each of the triple agonists; a negative control group not treated with a triple agonist and LPS; and a positive control group treated with only LPS without treatment with a triple agonist, were measured using a human TNF-$\alpha$ ELISA kit, and the results were compared (FIG. 12). For statistical treatment, the results were compared between the positive control group, the test groups, and the negative control group using a one-way ANOVA.

As a result, it was confirmed that the amount of human necrosis factor-$\alpha$ (TNF-$\alpha$), which was secreted in the media of all of the test groups, in which triple agonists that are capable of activating all of GLP-1, GIP, and glucagon receptors, without being limited to specific sequences, were treated on a human macrophage cell line, was significantly decreased compared to that in the positive control group treated with only LPS. From this result, the therapeutic effect of the triple agonists on liver disease accompanied by inflammation was confirmed.

Through the Examples above, the present inventors have confirmed that the long-acting conjugates of the triple agonists of the present invention can exhibit a therapeutic effect on various liver diseases, such as non-alcoholic steatohepatitis (NASH), fatty liver, primary biliary cirrhosis (PBC), and primary sclerosing cholangitis (PSC).

Taken together, the triple agonists of the present invention and long-acting conjugates thereof have a therapeutic effect on liver disease, and thus they can be effectively used in the manufacture of pharmaceutical drugs.

## Claims

1. A pharmaceutical composition for use in the prevention or treatment of liver disease, comprising:

   a pharmaceutically acceptable excipient; and
   a peptide comprising an amino acid sequence of SEQ ID NO: 42 in a pharmaceutically effective amount,
   wherein the liver disease is at least one disease selected from the group consisting of simple steatosis, non-alcoholic fatty liver (NAFL), liver inflammation, non-alcoholic steatohepatitis (NASH), metabolic liver disease, cholestatic liver disease, liver fibrosis, liver cirrhosis, liver decompensation, and liver cancer.

2. The pharmaceutical composition for use according to claim 1, wherein the peptide is in the form of a long-acting conjugate and the long-acting conjugate is represented by Formula 1 below:

   [Formula 1] X-L-F

   wherein in Formula 1 above,

   X is a peptide of an amino acid sequence of SEQ ID NO: 42;
   L is a linker comprising an ethylene glycol repeat unit;
   F is an immunoglobulin Fc fragment or a derivative thereof; and
   '-' represents a covalent bond between X and L and between L and F.

3. The pharmaceutical composition for use according to claim 1 or 2, wherein the C-terminus of the peptide is amidated.

4. The pharmaceutical composition for use according to claim 1 or 2, wherein the liver disease is liver inflammation.

5. The pharmaceutical composition for use according to claim 4, wherein the pharmaceutical composition reduces the expression of at least one of TNF-$\alpha$, MCP-1, and IL-6 in the liver tissue when administered.

6. The pharmaceutical composition for use according to claim 1 or 2, wherein the liver disease is a metabolic liver disease.

7. The pharmaceutical composition for use according to claim 6, wherein the pharmaceutical composition reduces the amount of triglycerides and/or cholesterol in the liver tissue when administered.

8. The pharmaceutical composition for use according to claim 1 or 2, wherein the liver disease is at least one disease selected from the group consisting of simple steatosis, non-alcoholic fatty liver (NAFL), liver inflammation, non-alcoholic steatohepatitis (NASH), cholestatic liver disease, liver fibrosis, cirrhosis, liver decompensation, and liver cancer.

9. The pharmaceutical composition for use according to claim 8, wherein the cholestasis liver disease is any one selected from the group consisting of primary biliary cirrhosis, primary sclerosing cholangitis, and a combination thereof.

10. The pharmaceutical composition for use according to claim 8, wherein the liver disease is non-alcoholic steatohepatitis (NASH) accompanying fatty liver, liver fibrosis, or cirrhosis.

11. The pharmaceutical composition for use according to claim 8, wherein the liver disease is liver cancer caused by non-alcoholic steatohepatitis (NASH).

12. The pharmaceutical composition for use according to claim 1 or 2, wherein the liver disease is at least one disease selected from the group consisting of simple steatosis, non-alcoholic fatty liver (NAFL), and cirrhosis.

**13.** The pharmaceutical composition for use according to claim 1 or 2, wherein the liver disease is at least one disease selected from the group consisting of liver inflammation, non-alcoholic steatohepatitis (NASH), and liver fibrosis.

**14.** The pharmaceutical composition for use according to claim 13, wherein the liver disease is liver fibrosis and the pharmaceutical composition reduces the blood concentration of TIMP-1 and/or hyaluronic acid in a subject administered with the pharmaceutical composition when administered.

**15.** The pharmaceutical composition for use according to claim 2, wherein the formula weight of the ethylene glycol repeat unit portion in the L is in the range of 1 kDa to 100 kDa.

## Patentansprüche

**1.** Pharmazeutische Zusammensetzung zur Verwendung bei der Vorbeugung oder Behandlung einer Lebererkrankung, die Folgendes umfasst:

einen pharmazeutisch annehmbaren Exzipienten und
ein Peptid, das eine Aminosäuresequenz der SEQ ID NO: 42 umfasst, in einer pharmazeutisch wirksamen Menge,
wobei die Lebererkrankung zumindest eine ist, die aus der aus einfacher Steatose, nicht-alkoholischer Fettleber (NAFL), Leberentzündung, nicht-alkoholischer Steatohepatitis (NASH), metabolischer Lebererkrankung, cholestatischer Lebererkrankung, Leberfibrose, Leberzirrhose, Leberdekompensation und Leberkrebs bestehenden Gruppe ausgewählt ist.

**2.** Pharmazeutische Zusammensetzung zur Verwendung nach Anspruch 1, wobei das Peptid in Form eines lang wirkenden Konjugats vorliegt und das lang wirkende Konjugat durch die nachstehende Formel 1 dargestellt ist:

[Formel 1] X-L-F

wobei in der obigen Formel 1:

X ein Peptid mit der Aminosäuresequenz der SEQ ID NO: 42 ist,
L ein Linker ist, der aus einer Ethylenglykol-Grundeinheit besteht,
F ein Immunglobulin-Fc-Fragment oder ein Derivat davon ist und
, - ' für eine kovalente Bindung zwischen X und L und zwischen L und F steht.

**3.** Pharmazeutische Zusammensetzung zur Verwendung nach Anspruch 1 oder 2, wobei der C-Terminus des Peptids amidiert ist.

**4.** Pharmazeutische Zusammensetzung zur Verwendung nach Anspruch 1 oder 2, wobei die Lebererkrankung eine Leberentzündung ist.

**5.** Pharmazeutische Zusammensetzung zur Verwendung nach Anspruch 4, wobei die pharmazeutische Zusammensetzung bei Verabreichung die Expression von zumindest einem aus TNF-$\alpha$, MCP-1 und IL-6 im Lebergewebe verringert.

**6.** Pharmazeutische Zusammensetzung zur Verwendung nach Anspruch 1 oder 2, wobei die Lebererkrankung eine metabolische Lebererkrankung ist.

**7.** Pharmazeutische Zusammensetzung zur Verwendung nach Anspruch 6, wobei die pharmazeutische Zusammensetzung bei Verabreichung die Menge an Triglyceriden und/oder Cholesterin im Lebergewebe verringert.

**8.** Pharmazeutische Zusammensetzung zur Verwendung nach Anspruch 1 oder 2, wobei die Lebererkrankung zumindest eine Erkrankung ist, die aus der aus einfacher Steatose, nicht-alkoholischer Fettleber (NAFL), Leberentzündung, nicht-alkoholischer Steatohepatitis (NASH), cholestatischer Lebererkrankung, Leberfibrose, Leberzirrhose, Leberdekompensation und Leberkrebs bestehenden Gruppe ausgewählt ist.

**9.** Pharmazeutische Zusammensetzung zur Verwendung nach Anspruch 8, wobei die Cholestase-Lebererkrankung

eine ist, die aus der aus primärer biliärer Zirrhose, primärer sklerosierender Cholangitis und einer Kombination davon bestehenden Gruppe ausgewählt ist.

**10.** Pharmazeutische Zusammensetzung zur Verwendung nach Anspruch 8, wobei die Lebererkrankung nichtalkoholische Steatohepatitis (NASH) ist, die zusammen mit Fettleber, Leberfibrose oder Zirrhose auftritt.

**11.** Pharmazeutische Zusammensetzung zur Verwendung nach Anspruch 8, wobei die Lebererkrankung Leberkrebs ist, der durch nichtalkoholische Steatohepatitis (NASH) verursacht wird.

**12.** Pharmazeutische Zusammensetzung zur Verwendung nach Anspruch 1 oder 2, wobei die Lebererkrankung zumindest eine Erkrankung ist, die aus der aus einfacher Steatose, nichtalkoholischer Fettleber (NAFL) und Zirrhose bestehenden Gruppe ausgewählt ist.

**13.** Pharmazeutische Zusammensetzung zur Verwendung nach Anspruch 1 oder 2, wobei die Lebererkrankung zumindest eine Erkrankung ist, die aus der aus Leberentzündung, nichtalkoholischer Steatohepatitis (NASH) und Leberfibrose bestehenden Gruppe ausgewählt ist.

**14.** Pharmazeutische Zusammensetzung zur Verwendung nach Anspruch 13, wobei die Lebererkrankung Leberfibrose ist und die pharmazeutische Zusammensetzung bei Verabreichung die Blutkonzentration von TIMP-1 und/oder Hyaluronsäure in einem Individuum, dem die pharmazeutische Zusammensetzung verabreicht wird, verringert.

**15.** Pharmazeutische Zusammensetzung zur Verwendung nach Anspruch 2, wobei das Formelgewicht des Ethylenglykol-Grundeinheitenabschnitts in L im Bereich von 1 kDa bis 100 kDa liegt.

## Revendications

**1.** Composition pharmaceutique à utiliser dans la prévention ou le traitement d'une maladie hépatique, comprenant :

un excipient pharmaceutiquement acceptable ; et
un peptide comprenant une séquence d'acides aminés de SEQ ID NO : 42 en une quantité pharmaceutiquement efficace,
dans laquelle la maladie hépatique est au moins une maladie choisie dans le groupe constitué de la stéatose simple, de la stéatose hépatique non alcoolique (NAFL), de l'inflammation du foie, de la stéatohépatite non alcoolique (NASH), de la maladie métabolique du foie, de la maladie hépatique cholestatique, de la fibrose du foie, de la cirrhose du foie, de la décompensation du foie et du cancer du foie.

**2.** Composition pharmaceutique à utiliser selon la revendication 1, dans laquelle le peptide est sous la forme d'un conjugué à longue durée d'action et le conjugué à longue durée d'action est représenté par la formule 1 ci-dessous :

[Formule 1] X - L - F

dans laquelle dans la formule 1 ci-dessus,

X est un peptide d'une séquence d'acides aminés de SEQ ID NO : 42 ;
L est un lieur comprenant une unité de répétition d'éthylène glycol ;
F est un fragment d'immunoglobuline Fc ou un dérivé de celui-ci ; et
« -« représente une liaison covalente entre X et L et entre L et F.

**3.** Composition pharmaceutique à utiliser selon la revendication 1 ou 2, dans laquelle l'extrémité C-terminale du peptide est amidée.

**4.** Composition pharmaceutique à utiliser selon la revendication 1 ou 2, dans laquelle la maladie hépatique est une inflammation du foie.

**5.** Composition pharmaceutique à utiliser selon la revendication 4, dans laquelle la composition pharmaceutique réduit l'expression d'au moins l'un de TNF-$\alpha$, MCP-1 et IL-6 dans le tissu hépatique lorsqu'elle est administrée.

6. Composition pharmaceutique à utiliser selon la revendication 1 ou 2, dans laquelle la maladie hépatique est une maladie métabolique du foie.

7. Composition pharmaceutique à utiliser selon la revendication 6, dans laquelle la composition pharmaceutique réduit la quantité de triglycérides et/ou de cholestérol dans le tissu hépatique lorsqu'elle est administrée.

8. Composition pharmaceutique à utiliser selon la revendication 1 ou 2, dans laquelle la maladie hépatique est au moins une maladie choisie dans le groupe comprenant la stéatose simple, la stéatose hépatique non alcoolique (NAFL) (NAFL), l'inflammation du foie, la stéatohépatite non alcoolique (NASH), la maladie hépatique cholestatique, la fibrose du foie, la cirrhose, la décompensation du foie et le cancer du foie.

9. Composition pharmaceutique à utiliser selon la revendication 8, dans laquelle la maladie hépatique cholestatique est une quelconque choisie dans le groupe constitué par une cirrhose biliaire primaire, une cholangite sclérosante primaire et une combinaison de celles-ci.

10. Composition pharmaceutique à utiliser selon la revendication 8, dans laquelle la maladie hépatique est une stéatohépatite non alcoolique (NASH) accompagnant une stéatose hépatique, une fibrose hépatique ou une cirrhose.

11. Composition pharmaceutique à utiliser selon la revendication 8, dans laquelle la maladie hépatique est un cancer du foie causé par une stéatohépatite non alcoolique (NASH).

12. Composition pharmaceutique à utiliser selon la revendication 1 ou 2, dans laquelle la maladie hépatique est au moins une maladie choisie dans le groupe constitué d'une stéatose simple, d'une stéatose hépatique non alcoolique (NAFL) et d'une cirrhose.

13. Composition pharmaceutique à utiliser selon la revendication 1 ou 2, dans laquelle la maladie hépatique est au moins une maladie choisie dans le groupe constitué d'une inflammation du foie, d'une stéatohépatite non alcoolique (NASH) et d'une fibrose du foie.

14. Composition pharmaceutique à utiliser selon la revendication 13, dans laquelle la maladie hépatique est une fibrose du foie et la composition pharmaceutique réduit la concentration sanguine de TIMP-1 et/ou d'acide hyaluronique chez un sujet après administration de la composition pharmaceutique lorsqu'elle est administrée.

15. Composition pharmaceutique à utiliser selon la revendication 2, dans laquelle le poids de formule de la partie d'unité de répétition d'éthylène glycol dans le L est dans la plage de 1 kDa à 100 kDa.

[FIG. 1]

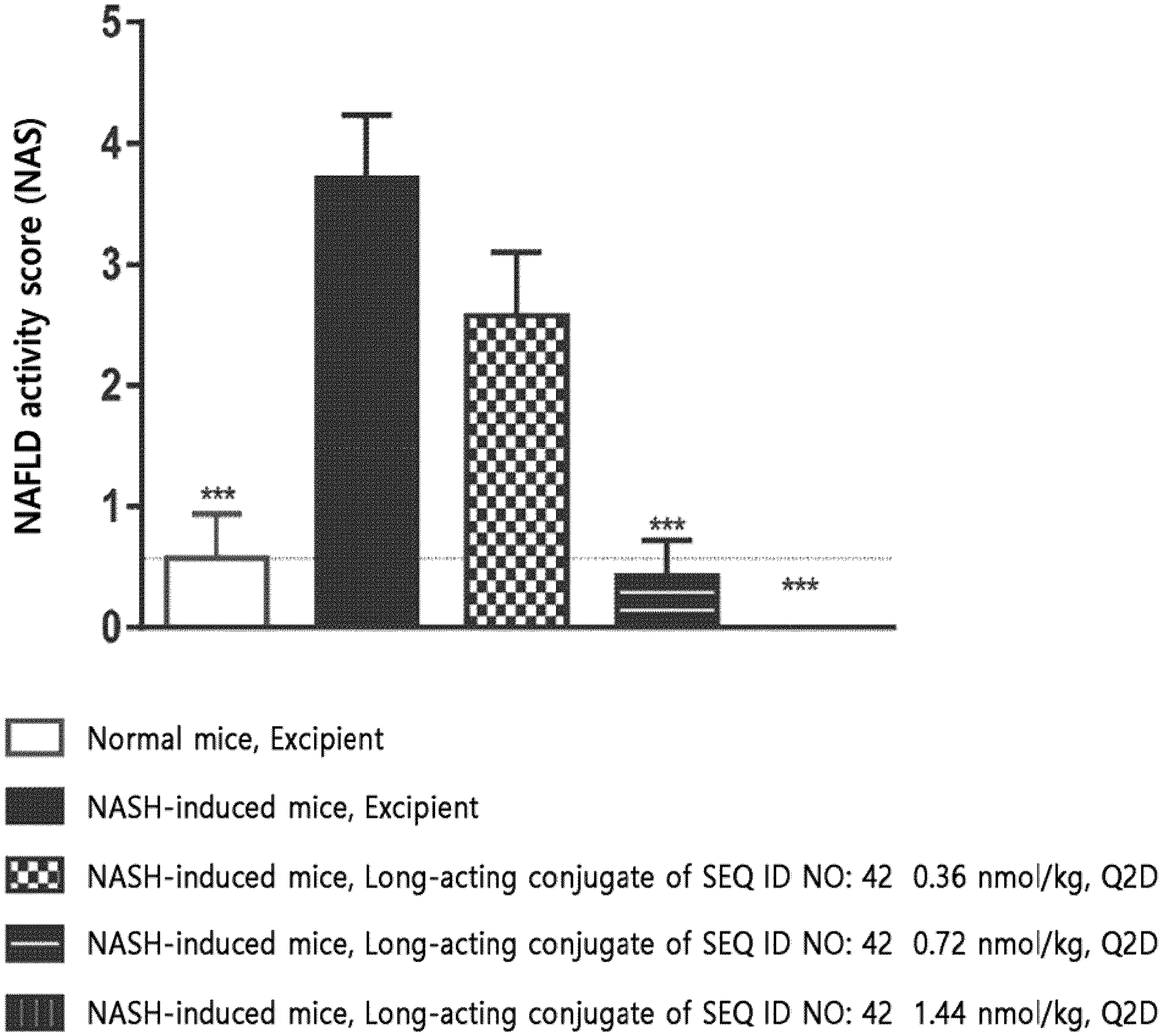
NAFLD activity score (NAS)
0
1
2
3
4
5
***
***
***
Normal mice, Excipient
NASH-induced mice, Excipient
NASH-induced mice, Long-acting conjugate of SEQ ID NO: 42 0.36 nmol/kg, Q2D
NASH-induced mice, Long-acting conjugate of SEQ ID NO: 42 0.72 nmol/kg, Q2D
NASH-induced mice, Long-acting conjugate of SEQ ID NO: 42 1.44 nmol/kg, Q2D

[FIG. 2]

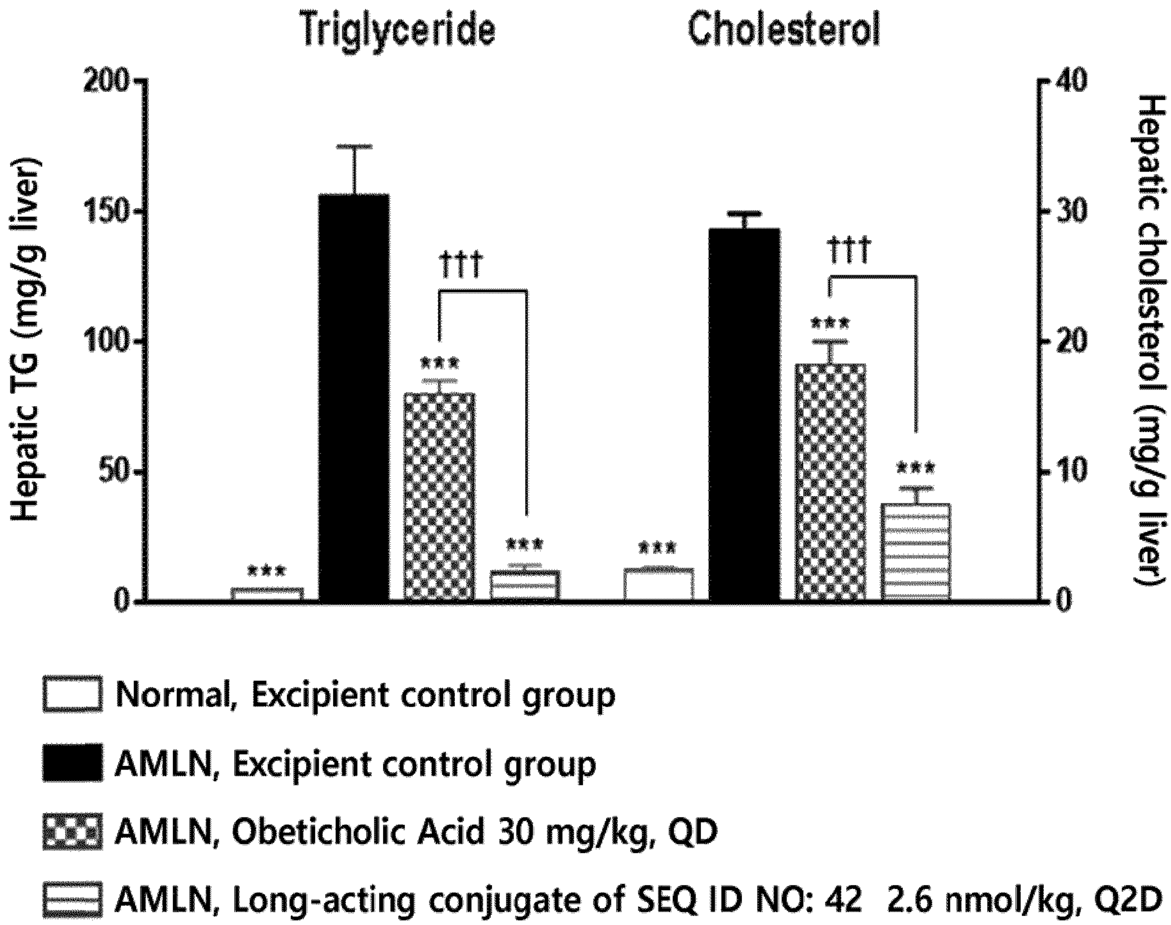
Triglyceride
Cholesterol
Hepatic TG (mg/g liver)
Hepatic cholesterol (mg/g liver)
200
150
100
50
0
40
30
20
10
0
***
†††
Normal, Excipient control group
AMLN, Excipient control group
AMLN, Obeticholic Acid 30 mg/kg, QD
AMLN, Long-acting conjugate of SEQ ID NO: 42 2.6 nmol/kg, Q2D

[FIG. 3]

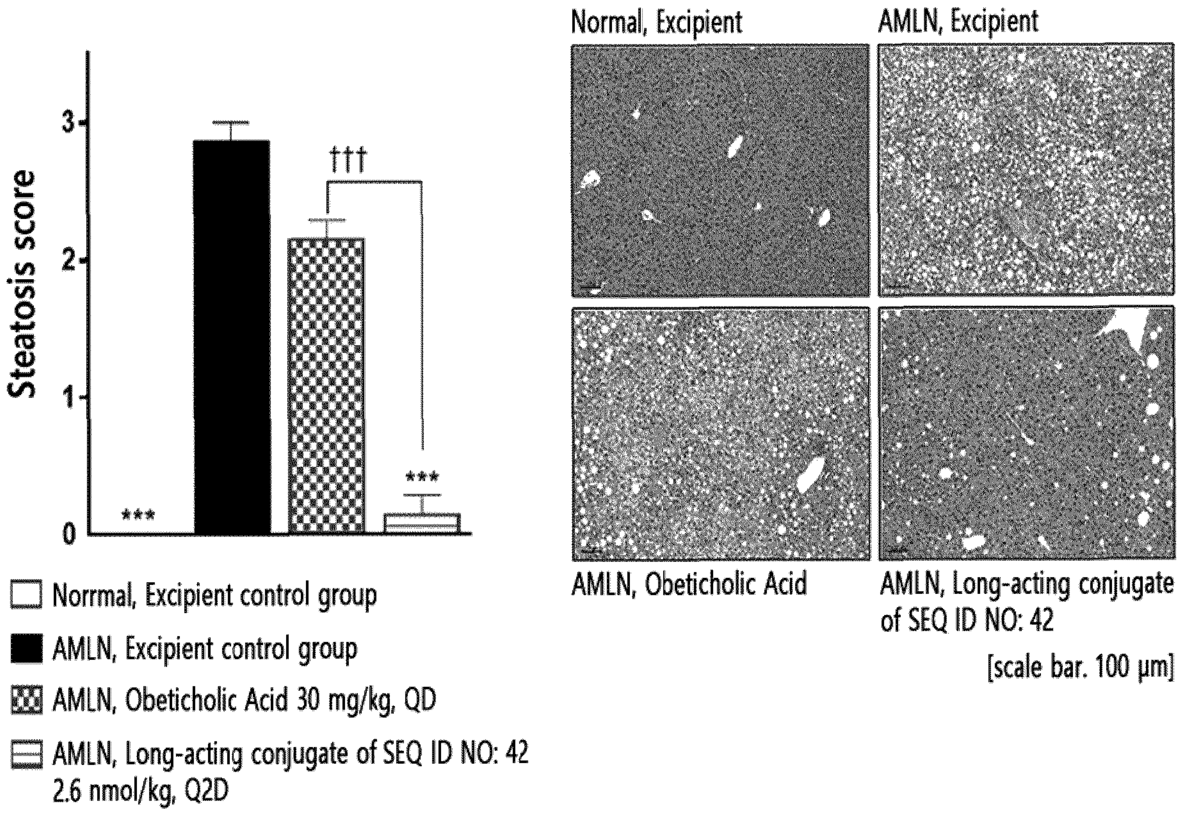
Steatosis score
3
2
1
0
***
†††
***
Norrmal, Excipient control group
AMLN, Excipient control group
AMLN, Obeticholic Acid 30 mg/kg, QD
AMLN, Long-acting conjugate of SEQ ID NO: 42
2.6 nmol/kg, Q2D
Normal, Excipient
AMLN, Excipient
AMLN, Obeticholic Acid
AMLN, Long-acting conjugate of SEQ ID NO: 42
[scale bar. 100 µm]

[FIG. 4]

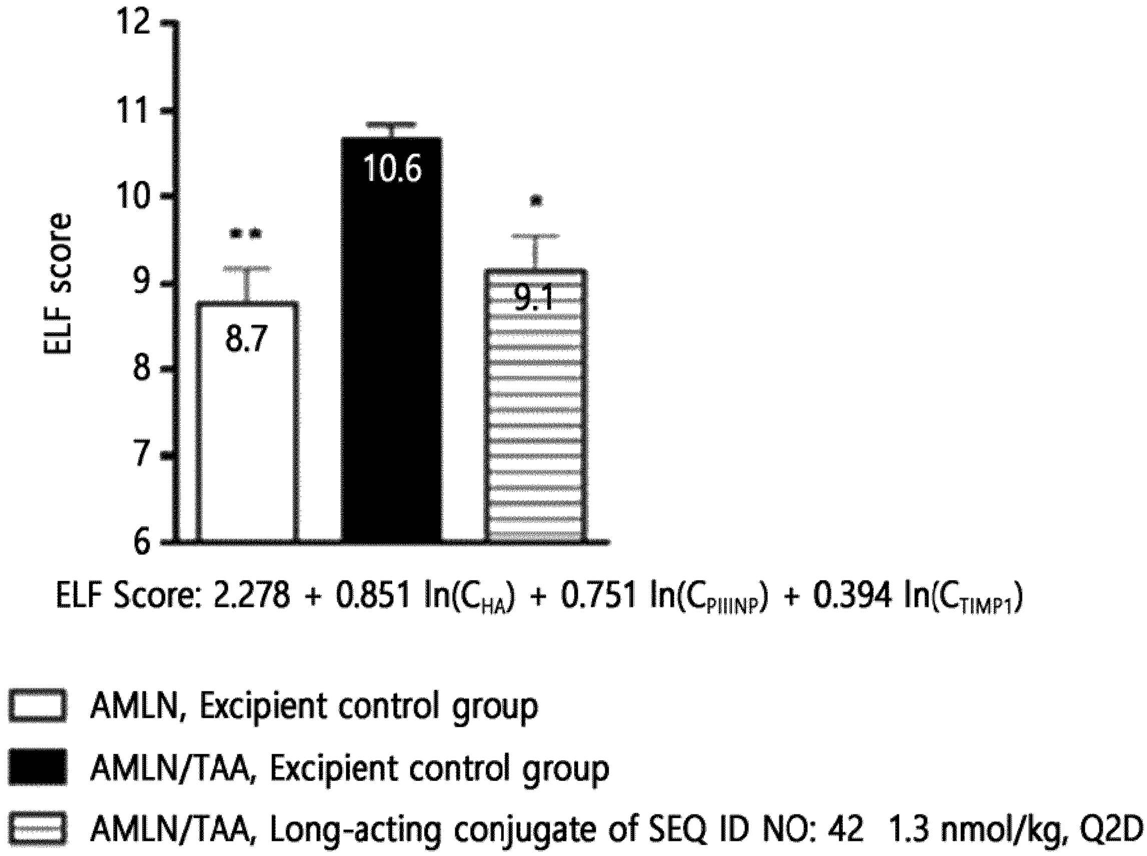
12
11
10
9
8
7
6
ELF score
**
*
10.6
8.7
9.1
ELF Score: 2.278 + 0.851 ln(C_HA) + 0.751 ln(C_PIIINP) + 0.394 ln(C_TIMP1)
AMLN, Excipient control group
AMLN/TAA, Excipient control group
AMLN/TAA, Long-acting conjugate of SEQ ID NO: 42 1.3 nmol/kg, Q2D

[FIG. 5]

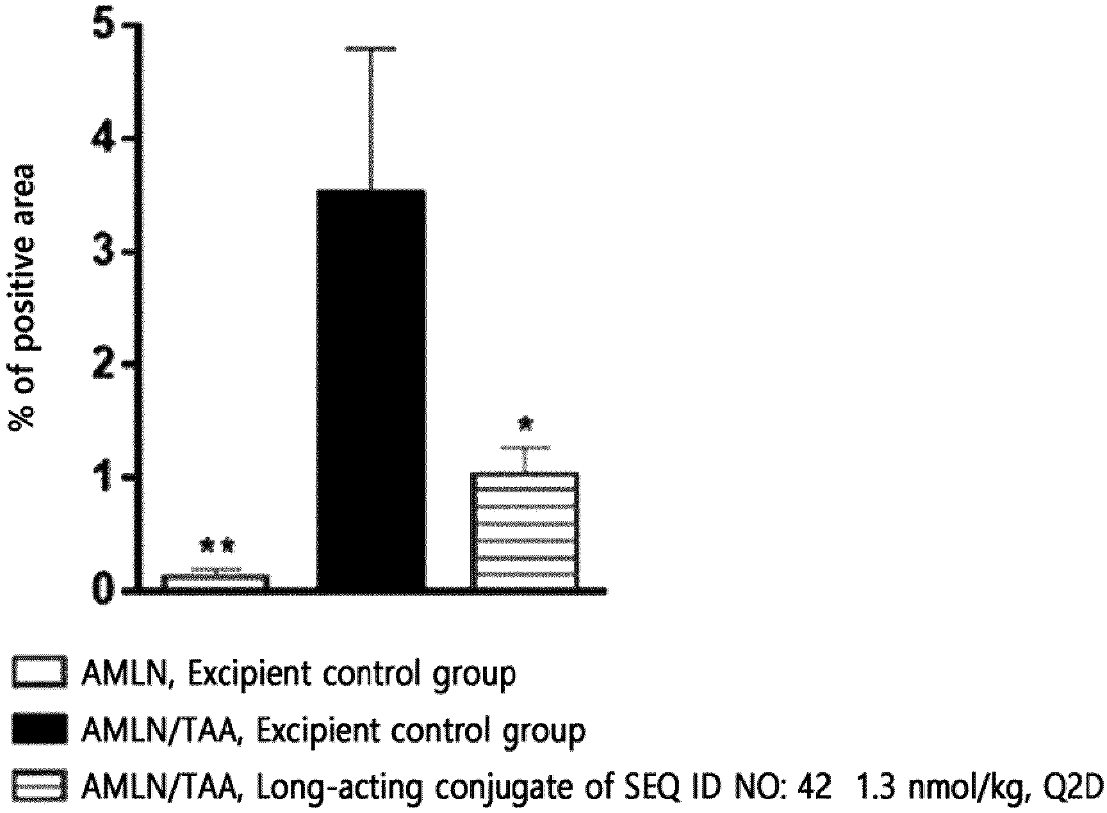
5
4
3
2
1
0
% of positive area
**
*
AMLN, Excipient control group
AMLN/TAA, Excipient control group
AMLN/TAA, Long-acting conjugate of SEQ ID NO: 42 1.3 nmol/kg, Q2D

[FIG. 6]

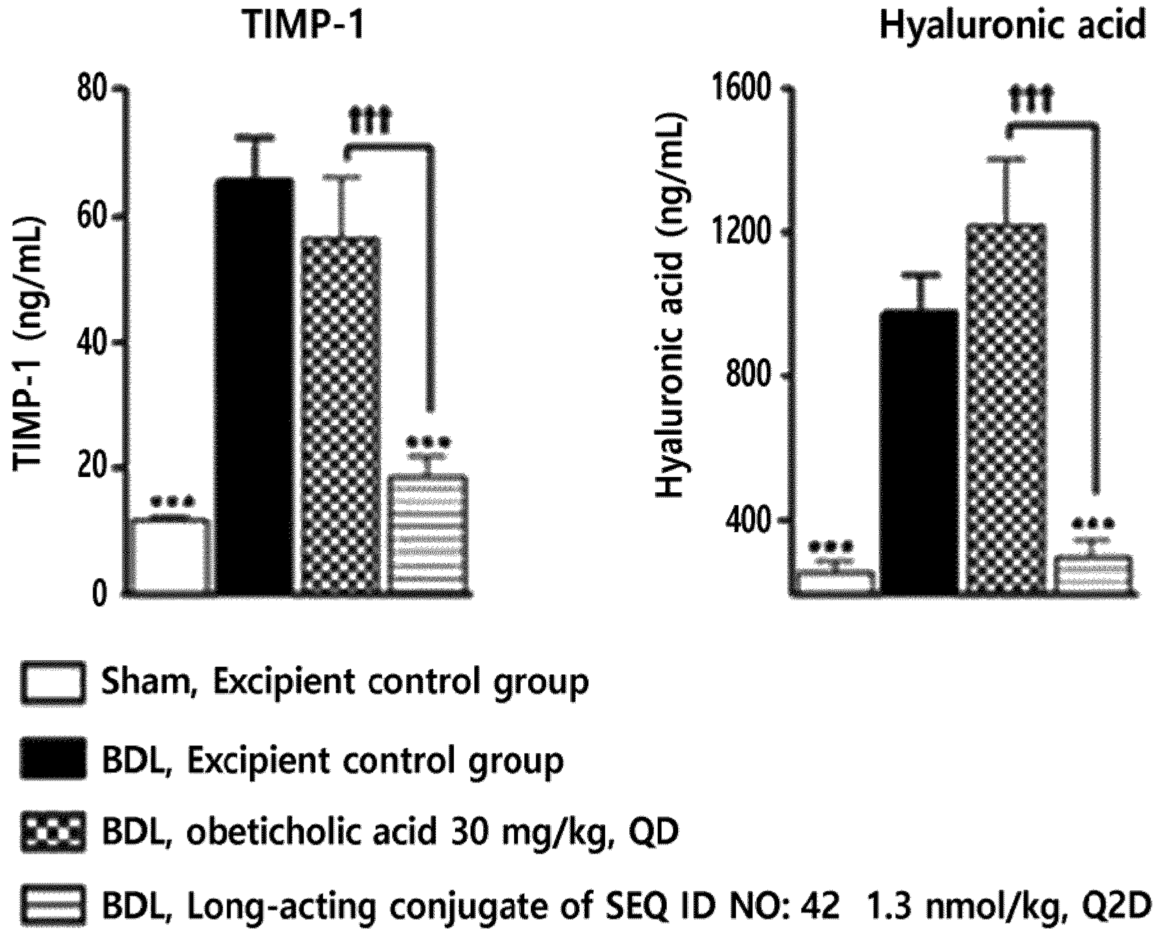
TIMP-1
TIMP-1 (ng/mL)
80
60
40
20
0
***
†††
***
Hyaluronic acid
Hyaluronic acid (ng/mL)
1600
1200
800
400
***
†††
***
Sham, Excipient control group
BDL, Excipient control group
BDL, obeticholic acid 30 mg/kg, QD
BDL, Long-acting conjugate of SEQ ID NO: 42 1.3 nmol/kg, Q2D

[FIG. 7a]

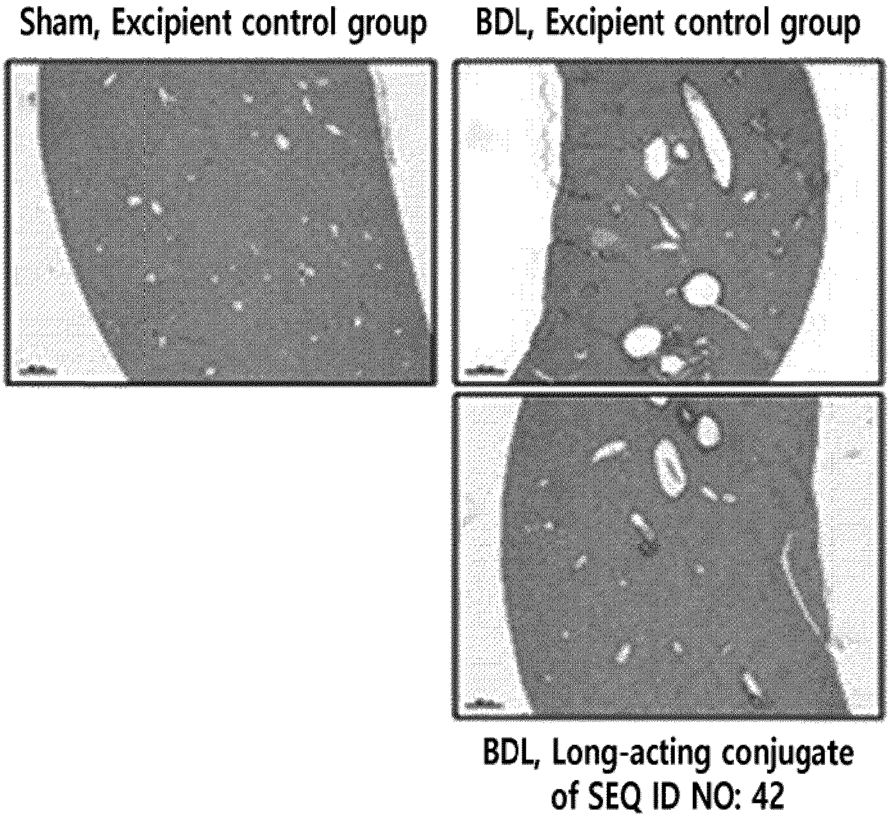
Sham, Excipient control group
BDL, Excipient control group
BDL, Long-acting conjugate of SEQ ID NO: 42

[FIG. 7b]

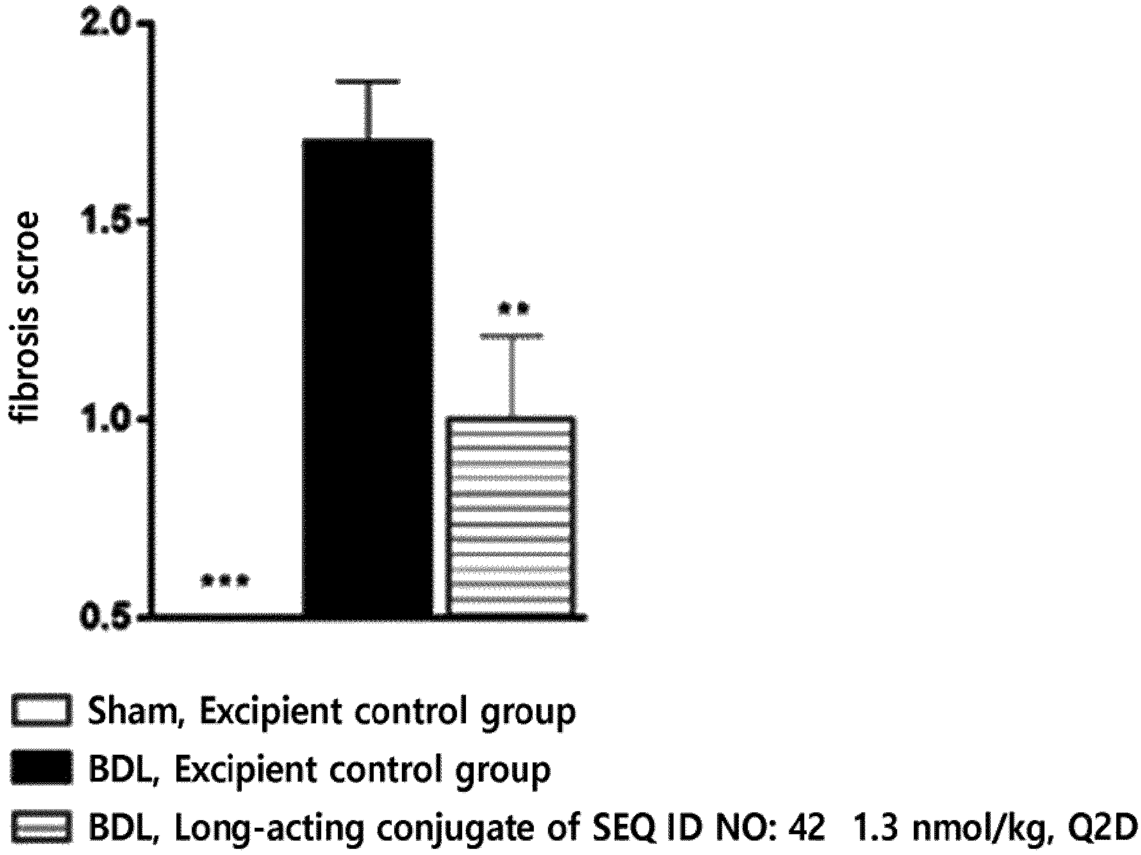
2.0
1.5
1.0
0.5
fibrosis scroe
***
**
Sham, Excipient control group
BDL, Excipient control group
BDL, Long-acting conjugate of SEQ ID NO: 42 1.3 nmol/kg, Q2D

[FIG. 8]

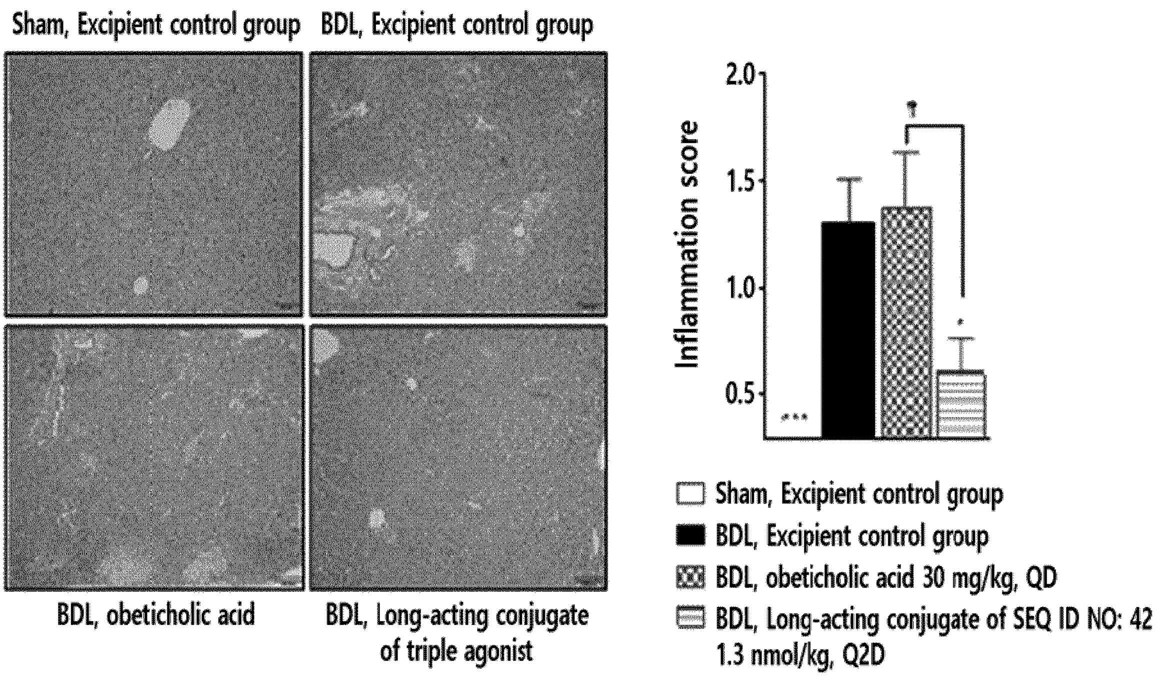
Sham, Excipient control group
BDL, Excipient control group
BDL, obeticholic acid
BDL, Long-acting conjugate of triple agonist
Inflammation score
2.0
1.5
1.0
0.5
***
†
*
Sham, Excipient control group
BDL, Excipient control group
BDL, obeticholic acid 30 mg/kg, QD
BDL, Long-acting conjugate of SEQ ID NO: 42 1.3 nmol/kg, Q2D

[FIG. 9]

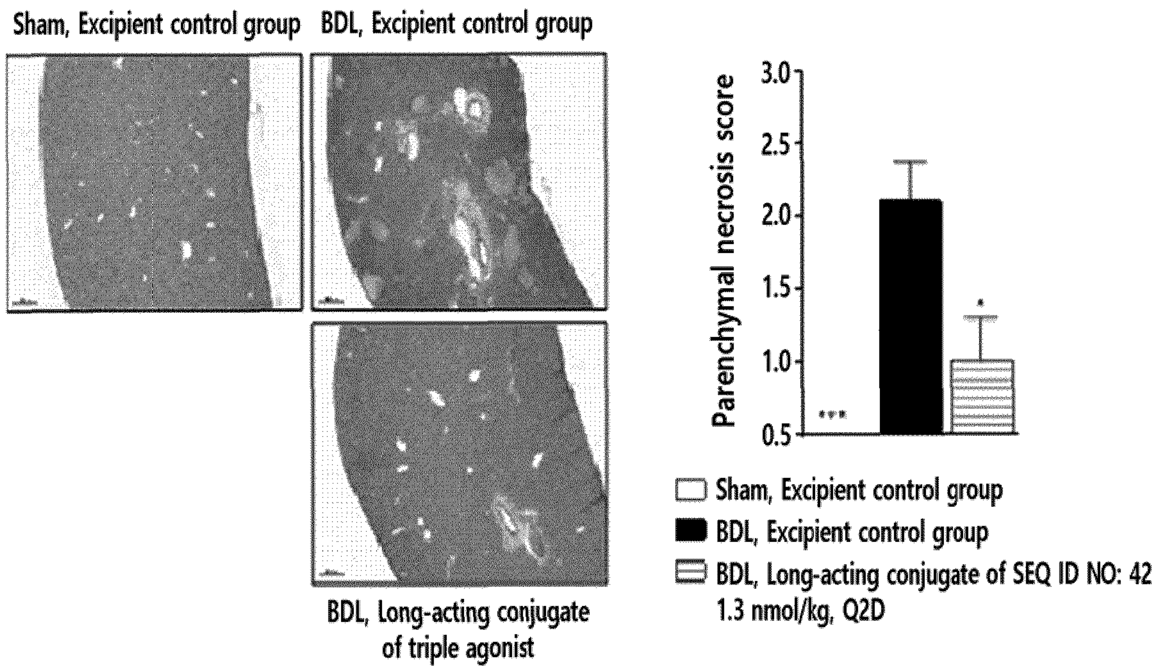
Sham, Excipient control group
BDL, Excipient control group
BDL, Long-acting conjugate of triple agonist
Parenchymal necrosis score
3.0
2.5
2.0
1.5
1.0
0.5
***
*
Sham, Excipient control group
BDL, Excipient control group
BDL, Long-acting conjugate of SEQ ID NO: 42 1.3 nmol/kg, Q2D

[FIG. 10]

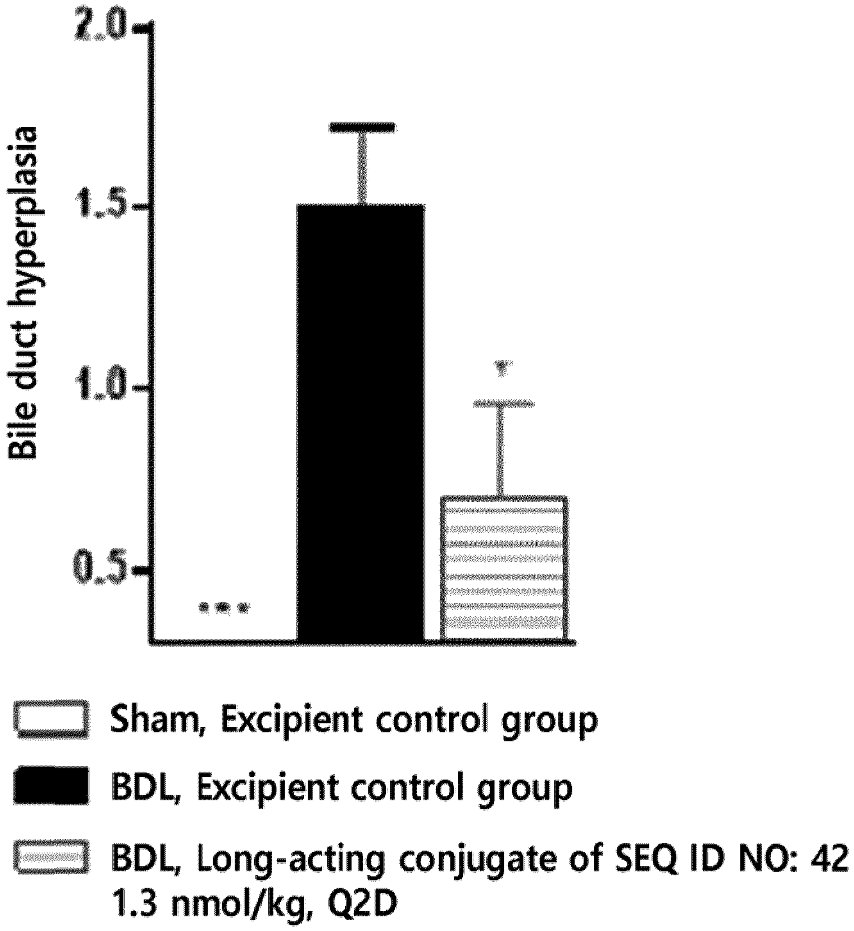
2.0
1.5
1.0
0.5
Bile duct hyperplasia
***
*
Sham, Excipient control group
BDL, Excipient control group
BDL, Long-acting conjugate of SEQ ID NO: 42
1.3 nmol/kg, Q2D

[FIG. 11]

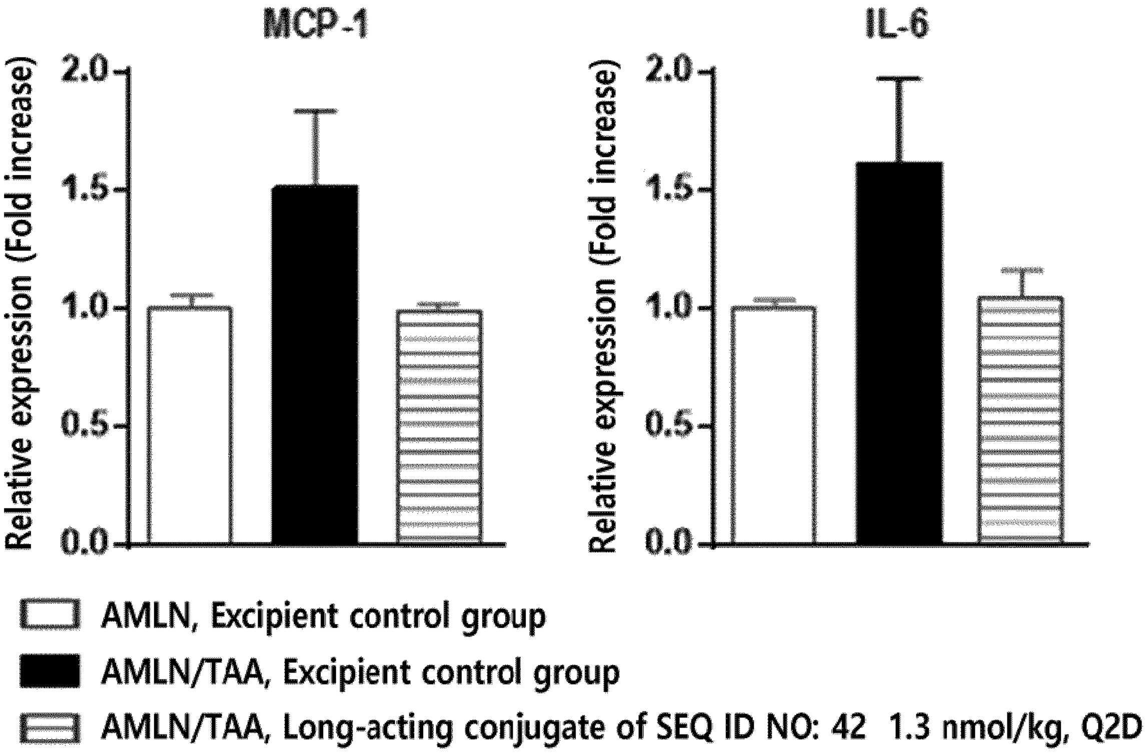
MCP-1
IL-6
Relative expression (Fold increase)
2.0
1.5
1.0
0.5
0.0
AMLN, Excipient control group
AMLN/TAA, Excipient control group
AMLN/TAA, Long-acting conjugate of SEQ ID NO: 42 1.3 nmol/kg, Q2D

[FIG. 12]

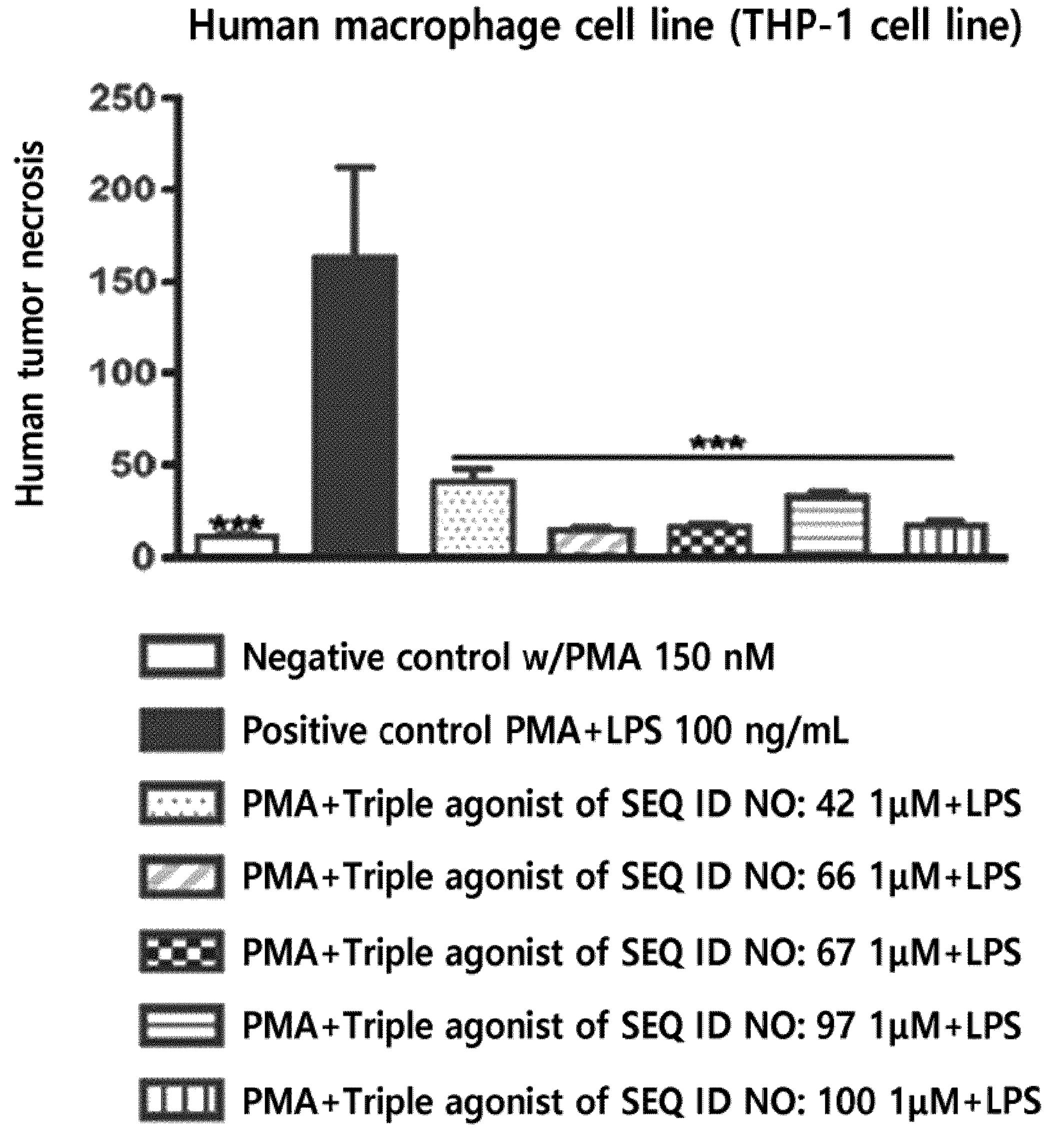
Human macrophage cell line (THP-1 cell line)
Human tumor necrosis
250
200
150
100
50
0
***
***
Negative control w/PMA 150 nM
Positive control PMA+LPS 100 ng/mL
PMA+Triple agonist of SEQ ID NO: 42 1µM+LPS
PMA+Triple agonist of SEQ ID NO: 66 1µM+LPS
PMA+Triple agonist of SEQ ID NO: 67 1µM+LPS
PMA+Triple agonist of SEQ ID NO: 97 1µM+LPS
PMA+Triple agonist of SEQ ID NO: 100 1µM+LPS

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2018311315 A1
- WO 9734631 A
- WO 9632478 A


### Non-patent literature cited in the description


- *JCI Insight.,* 2016, vol. 1 (11), e87336
- *Hepatology,* 2003, vol. 38, 1008-17
- *J Clin Invest,* 2001, vol. 108, 1167-74
- *Nat Rev Gastroenterol Hepatol,* March 2019, vol. 16 (3), 145-159
- **KIM et al.** *Diabetes,* 2019, vol. 68 (1), 990
- **CHOI et al.** *Diabetes,* 2017, vol. 66 (1), A304
- **CHOI et al.** *Diabetes,* 2018, vol. 67 (1), 1106
- **KIM et al.** *Diabetologia,* 2018, vol. 61 (1), S61-S62
- **H. NEURATH ; R. L. HILL.** The Proteins. Academic Press, 1979
- **G.J. WEBB et al.** *J. Autoimmunity,* November 2015, vol. 64, 42-52